# EUROPÄISCHE PATENTSCHRIFT

(11) **EP 1 311 625 B1**
(45) Veröffentlichungstag und Bekanntmachung des Hinweises auf die Patenterteilung: **01.02.2006**
(21) Anmeldenummer: 01971909.5
(22) Anmeldetag: 11.08.2001
(51) Int. Cl.: C09B 69/00

(54) **FUNKTIONALISIERTE PERYLENTETRACARBONSÄUREDIIMIDE**
FUNCTIONALIZED PERYLENE TETRACARBOXYLIC ACID DIIMIDES
DIIMIDES D'ACIDE TETRACARBOXYLIQUE DE PERYLENE FONCTIONNALISES

(30) Priorität: 14.08.2000 DE 10039643
(43) Veröffentlichungstag der Anmeldung: 21.05.2003
(73) Patentinhaber: Max-Planck-Gesellschaft zur Förderung der Wissenschaften e.V., 80539 München (DE)
(72) Erfinder: BECKER, Stefan, 68167 Mannheim (DE); KLOK, Harm-Anton, 55118 Mainz (DE); RODRIGUEZ-HERNANDEZ, Juan, 55116 Mainz (DE); SCHUCH, Falk, 65185 Wiesbaden (DE); MÜLLEN, Klaus, 50939 Köln (DE)
(74) Vertreter: Paashaus, Sabine
(86) Internationale Anmeldenummer: PCT/EP2001/009319
(87) Internationale Veröffentlichungsnummer: WO 2002/014414

(56) Entgegenhaltungen:
- WO-A-99/40123
- DATABASE CHEMABS [Online] 2001 retrieved from STN Database accession no. 135:92965 XP002192518 -& KLOK ET AL.: "Star-shaped fluorescent polypeptides" JOURNAL OF POLYMER SCIENCE., Bd. 39, Nr. 10, Februar 2001 (2001-02), Seiten 1572-1583, XP001061864 INTERSCIENCE PUBLISHERS., XX ISSN: 0887-624X

## Beschreibung

Die Erfindung betrifft die Verwendung von funktionalisierten Perylen-3,4,9,10-tetracarbonsäurediimiden als Initiator oder/und als Reaktionspartner für Polymerisationsreaktionen, unter Verwendung der Perylen-3,4,9,10-tetracarbonsäurediimid-Verbindungen hergestellte Polymere und die Verwendung dieser farbigen oder/und fluoreszierenden Polymere sowie neue funktionalisierte Perylentetracarbonsäurediimide.

Diimide der 3,4,9,10-Perylentetracarbonsäure sind Farbstoffe, die sich durch hohe Farbstärke, gute Lösungsmittelbeständigkeit, hohe Beständigkeit gegen Chemikalien und hervorragende thermische Stabilität auszeichnen. Weiterhin weisen diese Farbstoffe bzw. Pigmente eine hohe Deckkraft und eine hervorragende Licht- und Wetterbestätigkeit auf (Y. Nagao, Prog. Org. Coat. 31 (1-2) (1997), 43; K. Müllen et al., Polymer Materials Encyclopedia (Herausgeber J. C. Salamone) Vol. 7, CRC Press Inc., (1996), 4999).

Perylenderivate finden daher als Hochleistungsfarbstoffe/-pigmente für das Einfärben von technischen Kunststoffen, in hochwertigen Spritzlacken oder als Dispersionsfarben Verwendung (W. Herbst et al., Industrial Organic Pigments - Production, Properties, Applications, 2. Ausgabe, Wiley - VCH, Weinheim (1997)). Da insbesondere lösliche Perylentetracarbonsäurediimide oftmals eine starke Fluoreszenz mit Quantenausbeuten bis nahezu 100 % aufweisen, werden sie weiterhin als funktionelle Farbstoffe eingesetzt, z.B. in Fluoreszenzsolarkollektoren (G. Seybold et al., Dyes Pigm., 11 (1989), 303; M.J. Cook at al., Chem. Ber., 20 (1984), 914), als Laserfarbstoffe (R. Reisfeld et al., Chimia 44 (1990), 295; H.G. Löhmannsröben et al., Appl. Phys. B48 (1989), 449) oder in Gewächshausfolien (JP 62-132693; S. Haremsa, Chem. in unserer Zeit, 28 (1994), 233).

Perylentetracarbonsäurediimid-Verbindungen wurden beispielsweise verwendet, um chemolumineszente Zusammensetzungen herzustellen (US 5,705,103; US 5,597,517; US 5,281,367; US 5,122,306). Solche Zusammensetzungen werden durch das Vermischen eines Oxalats, eines Aktivators und eines Perylenderivats erhalten.

US 4,845,223 beschreibt Perylentetracarbonsäurediimid-Derivate, die als fluoreszierende Farbstoffe Verwendung finden. Diese Farbstoffe werden dem zu färbenden Material, beispielsweise einem Polymethylmethacrylat zugemischt. WO97/22607 beschreibt 1,7-disubstituierte Perylen-3,4,9,10-tetracarbonsäurediimide als Zwischenprodukte zur Herstellung der entsprechenden Dianhydride. WO99/40123 betrifft wässrige Polymerdispersionen, die Farbstoffe, unter anderem auch Perylenfarbstoffe enthalten. Die Verwendung von 2,9-dibutyl-5,6,12,13-tetra-[4-hydroxymethyl-phenoxy]-3,4,9,10-perylen-tetracarbonsäurediimid zur Herstellung eines elektrolumineszierenden Diodenmaterials wird in FR 2770222 offenbart.

Eine Aufgabe der vorliegenden Erfindung bestand darin, verbesserte Perylen-3,4,9,10-tetracarbonsäurediimid-Verbind-ungen bereitzustellen, sowie weitere Anwendungsfelder für diese Farbstoffe zu erschließen.

Diese Aufgabe wird erfindungsgemäß gelöst durch die Verwendung von Perylen-3,4,9,10-tetracarbonsäurediimid-Verbindungen der Formel (I) 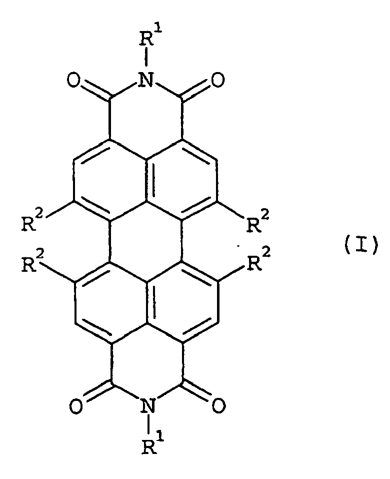
worin R¹ jeweils unabhängig eine Alkyl- oder Arylgruppe ist, die unverzweigt oder verzweigt oder/und nicht-substituiert oder substituiert sein kann, und
R² jeweils unabhängig H, Halogen, C₁-C₄-Alkyl, C₁-C₄-Alkoxy oder einen Rest darstellt, der mindestens eine funktionelle Gruppe Y enthält, ausgewählt aus Hydroxy, Ether, Ester, Halogen, Amin, Amid, Thiol, ethylenisch ungesättigter Doppelbindung, acetylenisch ungesättigter Dreifachbindung oder/und Carbonsäure, die gegebenenfalls mit einer Schutzgruppe oder einer Aktivierungsgruppe versehen ist, mit der Maßgabe, dass mindestens ein R² eine funktionelle Gruppe Y enthält,
als Initiator oder/und als Reaktionspartner für eine Polymerisationsreaktion.

Es wurde festgestellt, dass erfindungsgemäß funktionalisierte Perylentetracarbonsäurediimide als Initiator für unterschiedliche Polyreaktionen oder als Reaktionspartner in polymeranalogen Umsetzungen eingesetzt werden können. Gemeinsam ist allen für die hier vorgesehene Verwendung geeigneten Perylen-3,4,9,10-tetracarbonsäurediimidverbindungen, dass sie mindestens einen eine funktionelle Gruppe enthaltenden Rest an den Postitionen 1,6,7 oder/und 12 aufweisen, wobei die funktionelle Gruppe in einer Polymerisationsreaktion eine kovalente Bindung bildet. Solche funktionellen Gruppen können in freier Form, etwa als -OH, -NH₂, -SH, Halogen, insbesondere Br, als olefinische Doppelbindung (C=C), als Dreifachbindung (C=C) oder als -COOH vorliegen. Die funktionelle Gruppe Y kann aber auch in aktivierter Form, beispielsweise als Säureamid oder geschützt, beispielsweise als Ester, Amid, Carbamat oder Ether vorliegen. Geeignete Aktivierungsgruppen für Y = COOH sind Aktivester wie N-Hydroxysuccinimidylester (NHS-Ester), N-Hydroxybenzotriazolylester (HOBt Ester), Pentafluorphenylester (OPfp Ester), p-Nitrophenylester. Geeignete Aktivierungsgrupen für Y = OH sind Sulfonsäureester (z.B. Tosylat, Mesylat, Triflat), Carbonate (z.B. Benzotriazolcarbonat [BTC], p-Nitrophenylcarbonat [NPC], Glycidylether (Epoxide) und Carbonylimidazol (CDI).

Auf Grund der thermischen, chemischen und photochemischen Stabilität von Perylentetracarbonsäurediimidverbindungen können diese Chromophore verschiedene Polymerisationsreaktionsbedingungen überstehen, ohne ihren Farbstoffcharakter zu verlieren. So wurde festgestellt, dass die erfindungsgemäß eingesetzten Perylenverbindungen bei Reaktionsbedingungen, wie sie beispielsweise bei einer radikalischen Polymerisation, bei einer ringöffnenden Polymerisation von aliphatischen Polyestern, zyklischen Siloxanen oder N-Carboxyanhydriden und sogar bei hohen Temperaturen von Polykondensationsreaktionen stabil bleiben. Es ist deshalb möglich, die Perylenverbindungen als Initiator bzw. als Reaktionspartner bereits vor oder während der Polymerisationsreaktion zuzugeben und nicht erst später dem fertigen Polymerisat zuzumischen. Die Zugabe als Initiator oder Reaktionspartner bewirkt, dass die erfindungsgemäß eingesetzten Perylenverbindungen kovalent in die gebildeten Polymerisate eingebaut werden. Durch diese kovalente Bindung wird eine unerwünschte Migration bzw. Aggregation der Farbstoffe, wie sie beim bloßen Zumischen von Farbstoffen zu Polymeren auftreten können, in der Kunststoffmatrix verhindert. Die am Farbstoff kovalent angebrachten Polymerketten wirken dabei zusätzlich vorteilhaft als Compatibilizer mit der Matrix, machen also den Farbstoff mit der Matrix kompatibel, um so Entmischungserscheinungen zu verhindern. Darüber hinaus wird durch die kovalent mit dem Farbstoff verknüpften Polymerketten der Chromophor wirksam abgeschirmt. Diese Eigenschaft ist besonders für optische und optoelektronische Anwendungen von Polymeren vorteilhaft, weil die Fluoreszenzquantenausbeute durch diese Abschirmung auf hohem Niveau gehalten wird und jede unerwünschte Farbänderung durch Aggregation verhindert wird.

Der Ausdruck Initiator, wie hierin verwendet, bezeichnet ein Molekül, das eine Polymerisationsreaktion starten kann.

Der Ausdruck Reaktionspartner für eine Polymerisationsreaktion bezeichnet ein Molekül, welches in einer Polymerisationsreaktion umgesetzt und kovalent eingebaut wird und somit zum Wachstum der Polymerketten beiträgt.

Der Begriff Polymerisationsreaktion umfasst alle Reaktionen, die ein Kettenwachstum beinhalten. Beispiele für Polymerisationsreaktionen sind radikalische Polymerisationsreaktionen, Polyadditionen, Polykondensationen, anionische Polymerisationen, kationische Polymerisationen sowie koordinative Polymerisationen. Bei einer Polymerisationsreaktion oder Polyreaktion wird allgemein eine niedermolekulare Verbindung, beispielsweise ein Monomer oder Oligomer, in eine hochmolekulare Verbindung, etwa ein Polymer oder ein Polymerisat überführt.

Bei Verwendung als Inititator können die funktionalisierten Farbstoffe als Starter für Grafting from Polyreaktionen dienen. Beispiele für solche Polyreaktionen unter Verwendung eines funktionalisierten Perylenfarbstoffes sind die Synthese von Polyestern, wie etwa Polycaprolactonen, Polylactiden oder Polyamiden, insbesondere Nylon, von Polypeptiden, z.B. durch Polymerisation von Aminosäure-N-Carboxyanhydriden, von Polysiloxanen, von Polystyrolen oder Poly(meth)acrylaten. Bei Verwendung von Perylenverbindungen, die zwei funktionelle Gruppen Y enthalten, sind auf diese Weise lineare Polymere zugänglich, bei Verwendung von Perylenfarbstoffen mit drei oder mehr funktionellen Gruppen Y werden Sternpolymere erhalten.

In einer weiteren bevorzugten Ausführungsform werden die funktionalisierten Perylenfarbstoffe in Grafting onto Reaktionen verwendet, wobei geeignet funktionalisierte Polymerketten, wie etwa Polyalkylenoxide, insbesondere Polyethylenglykol, Polystyrole oder Polypeptide kovalent mit den Perylenfarbstoffen verknüpft werden.

Die funktionalisierten Perylenfarbstoffe können auch als Reaktionspartner, insbesondere als Comonomere, mit anderen Monomeren copolymerisiert werden, um dadurch einen polymeren Farbstoff bzw. ein gefärbtes Polymer zu bilden. Auf diese Weise können beispielsweise gefärbte Polyurethane, Polyparaphenylene, Polyfluorene oder Polystilbene hergestellt werden. Bei Verwendung von Perylenverbindungen mit drei oder mehr funktionellen Gruppen Y, können diese dabei zusätzlich als Vernetzer wirken.

Die funktionalisierten Perylenfarbstoffe können weiterhin auch als Reaktivfarbstoffe zur Einfärbung von Polymeren, insbesondere von technischen Polymeren verwendet werden. Dazu werden die funktionalisierten Farbstoffe zusammen mit geeigneten Monomeren und gegebenenfalls Hilfsstoffen einer polymeranalogen Reaktion, beispielsweise einer Umesterung unterworfen, wobei die funktionalisierten Perylenfarbstoffe kovalent in das Polymergrundgerüst eingebaut werden. Die Farbstoffe können dabei kovalent sowohl in die Polymerhauptkette als auch in oder als Seitenkette gebunden werden. Besonders vorteilhaft ist es, die Umsetzung in einem Extruder durchzuführen. Falls funktionalisierte Perylenderivate mit drei oder mehr funktionellen Gruppen Y eingesetzt werden, können sie auch hier zusätzlich die Funktion eines Vernetzers übernehmen.

Die Perylentetracarbonsäurediimidfluorophore weisen bevorzugt eine für viele Anwendungen, insbesondere biowissenschaftliche Anwendungen günstige langwellige, rote Fluoreszenz auf. Bevorzugt haben die erfindungsgemäß eingesetzten funktionalisierten Perylenfarbstoffe eine Fluoreszenz bei einer Wellenlänge > 500 nm und besonders bevorzugt bei einer Wellenlänge > 550 nm.

Die erfindungsgemäß eingesetzten Farbstoffe weisen mindestens einen Rest R² auf, der eine funktionelle Gruppe Y enthält, die einen kovalenten Einbau der Farbstoffe in Polymermoleküle ermöglicht. Die übrigen Reste R² werden aus Einheiten ausgewählt, die unter den Polymerisationsreaktionsbedingungen inert sind, wie etwa H, Halogen, insbesondere Br oder Cl, C₁-C₄-Alkyl, C₁-C₄-Alkoxy,

C₅-C₁₅-Aryl und C₅-C₁₅-Aryloxy, insbesondere Phenoxy. Die Arylreste oder Aryloxyreste können ihrerseits wiederum mit einem inerten Rest, wie etwa C₁-C₄-Alkyl, substituiert sein. Bei den Resten R², die mindestens eine funktionelle Gruppe Y enthalten, kann es sich grundsätzlich um beliebige organische Reste handeln. Bevorzugt handelt es sich um einen mit mindestens einer funktionellen Gruppe Y substituierten Alkyl- oder Arylrest, der unverzweigt oder verzweigt sein kann und gegebenenfalls weitere Substituenten aufweisen kann. Geeignete Reste, die mit einer funktionellen Gruppe Y substitutiert sein können, sind beispielsweise C₁-C₃₀-Alkylreste, C₁-C₃₀-Alkoxyreste, C₁-C₃₀-Alkylthioreste, C₅-C₃₀-Arylreste und bevorzugt C₅-C₃₀-Aryloxy- oder Arylthioreste. Die Reste R² können neben der funktionellen Gruppe Y weitere inerte Substituenten aufweisen, wie etwa C₁-C₃₀-Alkylreste. Bei der funktionellen Gruppe Y handelt es sich bevorzugt um -OH, -OR (worin R ein C₁-C₃₀-Kohlenwasserstoffrest oder ein Silylrest ist, der unsubstituiert oder substituiert sein kann, insbesondere ein unsubstituierter oder substituierter Methyl-, Benzyl-, Ethyl- oder ein substituierter Silylrest), O (C=O) (CH₂) ₙHal, NH₂, NHBoc, NHCBZ, NH(C=O) (CH₂)ₙHal, O (C=O) (CH₂),CH₃ oder COOH (worin n eine Zahl von 1 bis 30, bevorzugt von 1 bis 8 ist).

Besonders bevorzugt weist mindestens ein Rest R² die Struktur 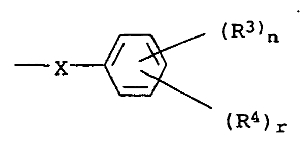 auf, worin X O oder S darstellt,
n eine ganze Zahl von 1 bis 5 ist, r eine ganze Zahl von 0 bis 5 ist, mit der Maßgabe n + r ≤ 5, R³ jeweils unabhängig eine Alkyl- oder Arylgruppe ist, die unverzweigt oder verzweigt, unsubstituiert oder substituiert sein kann und mindestens eine funktionelle Gruppe Y als Substituent aufweist und R⁴ C₁-C₄-Alkyl bedeutet. Beispiele für besonders bevorzugte Reste R₂ sind 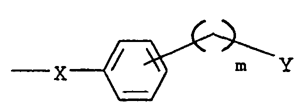 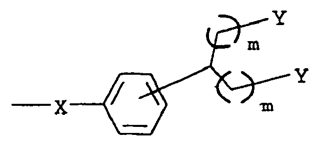
worin X und Y die oben angegebene Deutung haben und m bei jedem Auftreten jeweils unabhängig eine ganze Zahl von 1 bis 30, bevorzugt 1 bis 8 und besonders bevorzugt 1 oder 2 darstellt.

Erfindungsgemäß besonders bevorzugt sind Perylenderivate, die mindestens zwei funktionelle Gruppen Y oder mindestens vier funktionelle Gruppen Y enthalten. Verbindungen mit zwei funktionellen Gruppen Y können z.B. als Initiator zur Herstellung von linearen Polymeren oder als Comonomer zur Kettenverlängerung eingesetzt werden. Verbindungen mit vier funktionellen Gruppen Y können z.B. zur Herstellung von sternförmigen Polymeren mit vier Armen oder als Vernetzer eingesetzt werden.

Der an das Stickstoffatom der Carbonsäureimidgruppierung gebundene Rest R¹ ist jeweils unabhängig eine Alkyl- oder Arylgruppe, die unverzweigt oder verzweigt, unsubstituiert oder substituiert sein kann. Bevorzugt ist der Rest R¹ eine C₁-C₃₀-, insbesondere eine C₁-C₈- und besonders bevorzugt eine C₁-C₄-Alkylgruppe. Es wurde festgestellt, dass Verbindungen, in denen mindestens ein Rest R¹ eine aromatische Gruppe umfasst, beispielsweise eine C₅-C₃₀-Arylgruppe, insbesondere eine C₅-C₁₀-Arylgruppe, welche gegebenenfalls auch Heteroatome, wie etwa N, O oder S enthalten kann, eine besonders hohe Stabilität aufweisen. Geeignete Substituenten für R¹ sind beispielsweise inerte Substituenten, wie etwa Kohlenwasserstoffreste mit 1 bis 30 C-Atomen, insbesondere C₁-C₄-Alkyl oder funktionelle Gruppen, insbesondere die oben definierte Gruppe Y. Während der Rest R¹ derart ausgewählt sein kann, dass er unter den Polymerisationsreaktionsbedingungen inert ist, d.h. nicht an der Polymersationsreaktion teilnimmt, ist es für viele Anwendungen vorteilhaft, wenn auch mindestens ein Rest R¹ eine oder mehrere funktionelle Gruppen Y enthält. Auf diese Weise kann die Wertigkeit der funktionellen Farbstoffe weiter erhöht werden. Durch geeignete Wahl von Substituenten, in denen z.B. vier funktionelle Gruppen Y in den Resten R² und zwei weitere funktionelle Gruppen in den Resten R¹ vorliegen, kann somit beispielsweise ein sechswertiger funktionalisierter Farbstoff erhalten werden.

Bevorzugt ist R¹ ein Phenylrest der Formel 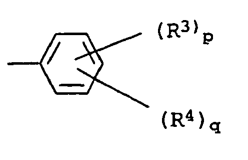
worin R⁴ C₁-C₄-Alkyl, also insbesondere Methyl, Ethyl, Propyl, Isopropyl, Butyl, Isobutyl oder tert.-Butyl darstellt, R₃ die oben angegebene Bedeutung hat und p und q jeweils eine ganze Zahl von 0 bis 5 darstellen, mit der Maßgabe, dass p + q ≤ 5.

Beispiele für besonders bevorzugte Reste R¹ sind

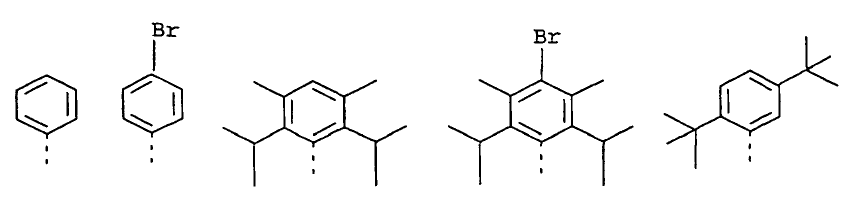

Für den Fall, dass der Rest R¹ eine funktionelle Gruppe enthält, weist er bevorzugt die Formel 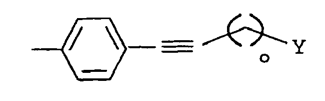 auf, worin o eine ganze Zahl von 1 bis 30, bevorzugt von 1 bis 8 und besonders bevorzugt 1 oder 2 darstellt.

Ein weiterer Gegenstand der Erfindung sind farbige oder/und fluoreszierende Polymere, die unter Verwendung einer Perylenverbindung als Initiator oder/und als Reaktionspartner für eine Polymerisationsreaktion, wie oben beschrieben, erhalten werden können. Die erfindungsgemäßen farbigen oder/und fluoreszierenden Polymere enthalten kovalent gebunden ein Perylenchromophor und werden aus den oben beschriebenen funktionalisierten Perylenverbindungen und mindestens einem Monomer gebildet. Beispiele für geeignete Monomere sind Verbindungen mit einer polymerisierbaren Gruppe, die mit der funktionellen Gruppe Y umgesetzt werden kann. Besonders bevorzugt sind Styrol, Acrylsäure, Acrylsäureester und -amide, Methacrylsäure, Methacrylsäureester und -amide, Oxazoline, cyclische Olefine (z.B. Norbonen), α,ω-Diolefine, Organosiliciumverbindungen (insbesondere Cyclosiloxane), Aminosäuren (besonders in Form der N-Carboxanhydride), Peptide, Ether, insbesondere cyclische Ether wie z.B. Ethylenoxid, Ester, Caprolactone, Lactide etc. Da der Farbstoff in den erfindungsgemäßen Polymeren kovalent eingebunden ist, kann keine Entmischung, Aggregation oder Migration des Farbstoffes innerhalb der Kunststoffmatrix auftreten, und es werden Polymere mit gleichbleibenden und gleichmäßigen Farbeigenschaften erhalten.

Durch Anbringen wasserlöslicher Polymerketten, beispielsweise von Polypeptiden oder Polyalkylenoxiden, insbesondere Polyethylenoxid, an die funktionalisierten Perylentetracarbonsäurediimide können auch wasserlösliche fluoreszente Stoffe erhalten werden. Diese wasserlöslichen fluoreszenten Stoffe sind überraschenderweise auch in wässriger Lösung oder in Wasser fluoreszierend, während unfunktionalisierte Perylentetracarbonsäurediimide in Wasser üblicherweise weder löslich noch fluoreszierend sind. Ein besonders bevorzugtes Beispiel für solche Stoffe sind sternförmige Polypeptide, die eine starke Fluoreszenz auch in wässriger Lösung zeigen.

Eine Fluoreszenzmarkierung mit den erfindungsgemäßen Polymeren bringt zudem den Vorteil einer leichten Detektierbarkeit mit sich, da Fluoreszenz empfindlich, bis hin zur Fluoreszenz eines einzelnen Moleküls gemessen werden kann. Dadurch erschließen sich besonders wirkungsvolle Anwendungen in den Biowissenschaften. Perylentetracarbonsäurediimide, die beispielsweise mit Polypeptid-, Polylactid- oder Polycaprolactonketten kovalent verbunden sind, können z.B. als Fluoreszenzlabels für Drug-Delivery- oder Drug-Release-Systeme verwendet werden, deren Verhalten dann in Tests in vitro oder im Organismus anhand der Fluoreszenz leicht beobachtet werden kann. Besonders vorteilhaft ist hierbei die Verwendung von Perylentetracarbonsäurediimidchromophoren, die eine langwellige, rote Fluoreszenz aufweisen, insbesondere bei einer Emissionswellenlänge > 500 nm. In diesem Spektralbereich ist praktisch keine störende Hintergrundfluoreszenz in biologischen Proben zu beobachten.

Die Erfindung betrifft weiterhin die oben vorgestellten funktionalisierten Perylentetracarbonsäurediimidverbindungen, welche mindestens eine Gruppe aufweisen, die ihre kovalente Verknüpfung mit Monomeren bzw. Polymeren ermöglicht. Insbesondere betrifft die Erfindung auch Perylen-3,4,9,10-tetracarbonsäurediimid-Verbindungen der Formel (I) 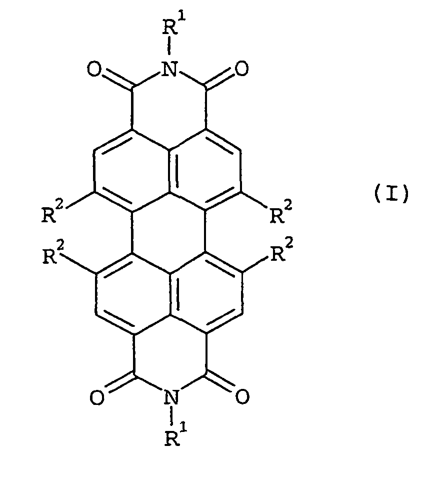
worin R¹ jeweils unabhängig einen Arylrest darstellt, der gegebenenfalls substituiert sein kann, und
R² jeweils unabhängig H, Halogen, C₁-C₄-Alkyl, C₁-C₄-Alkoxy, C₅-C₁₅-Aryl , C₅-C₁₅-Aryloxy oder einen Rest darstellt, der mindestens eine funktionelle Gruppe Y enthält, ausgewählt aus Hydroxy, Ether, Ester, Amin, Amid, Thiol, ethylenisch ungesättigter Doppelbindung, acetylenisch ungesättigter Dreifachbindung oder/und Carbonsäure, die gegebenenfalls mit einer Schutzgruppe oder einer Aktivierungsgruppe versehen ist, mit der Maßgabe, dass insgesamt drei oder mehr funktionelle Gruppen Y in den Resten R₂ vorhanden sind.

Bevorzugte Ausführungsformen der Reste R¹ und R² sind wie oben beschrieben.

Weiterhin bevorzugt sind Perylen-3,4,9,10-tetracarbonsäurediimid-Verbindungen der Formel (I), in denen einer oder beide Reste R¹ mindestens einen Br-Substituenten enthalten und R² jeweils unabhängig H, Halogen, C₁-C₄-Alkyl, C₁-C₄-Alkoxy, C₅-C₁₅-Aryl, C₅-C₁₅-Aryloxy oder einen Rest darstellt, der mindestens eine funktionelle Gruppe Y enthält, ausgewählt aus Hydroxy, Ether, Ester, Halogen, Amin, Amid, Thiol, ethylenisch ungesättigter Doppelbindung, acetylenisch ungesättigter Dreifachbindung oder/und Carbonsäure, die gegebenenfalls mit einer Schutzgruppe oder einer Aktivierungsgruppe versehen ist, mit der Maßgabe, dass mindestens ein R² eine funktionelle Gruppe Y enthält. Solche Verbindungen sind insbesondere als Zwischenprodukte zur Herstellung von mehrfach funktionalisierten Perylenverbindungen geeignet, die dann in der oben beschriebenen Weise kovalent in Polymerketten eingebaut werden können.

Die oben beschriebenen funktionalisierten Perylen-3,4,9,10-tetracarbonsäurediimidverbindungen können mit dem Fachmann bekannten Reaktionen erhalten werden.

Die Erfindung wird durch die beigefügten Figuren und die nachfolgenden Beispiele weiter erläutert.

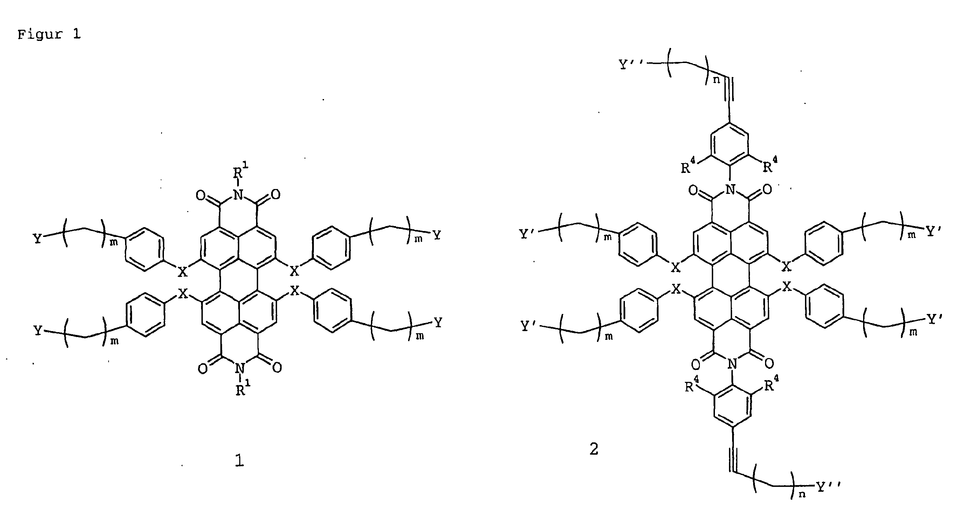

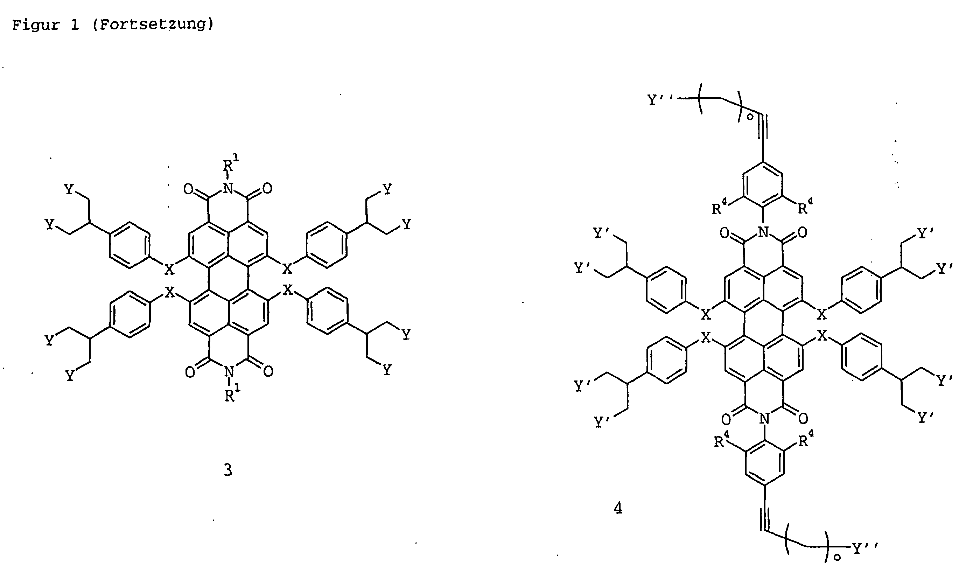

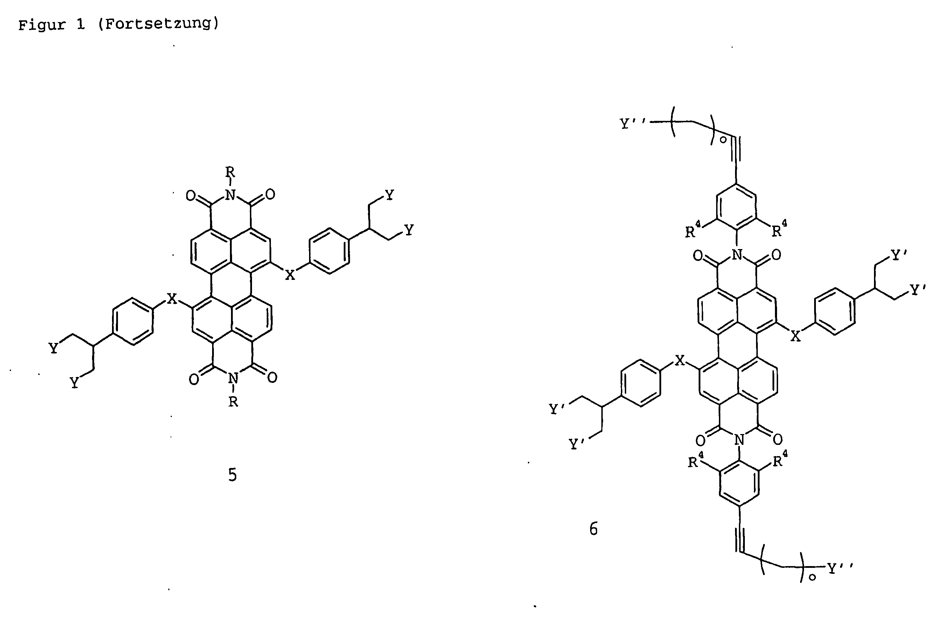

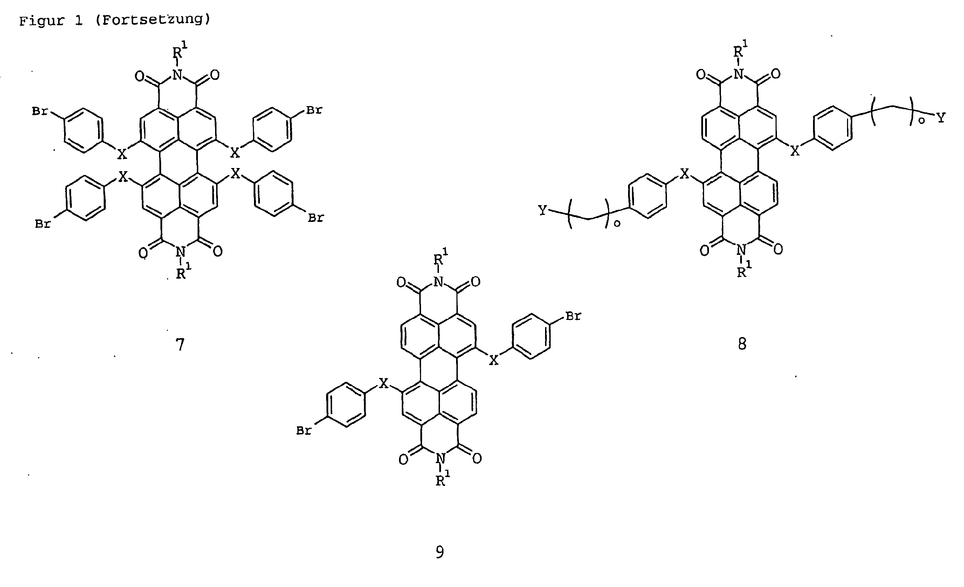

- Figur 1: zeigt bevorzugte Strukturen der erfindungsgemäßen funktionalisierten Perylentetracarbonsäurediimide.

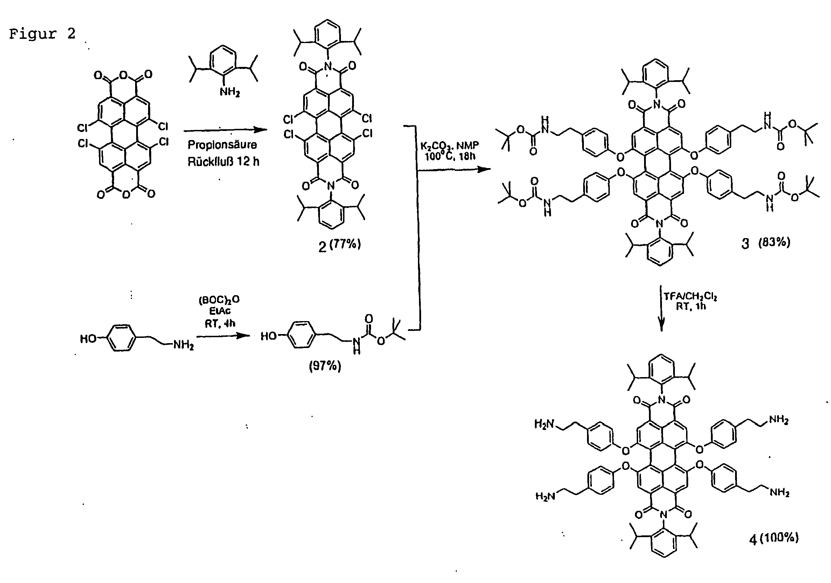

- Figur 2: zeigt einen Syntheseweg zur Herstellung eines vierfach mit primären Amingruppen funktionalisierten Perylentetracarbonsäurediimids.

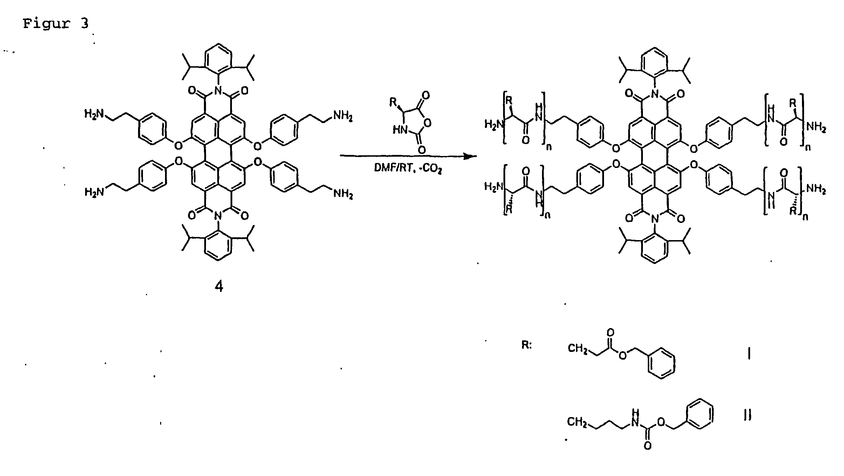

- Figur 3: zeigt einen Syntheseweg zur Herstellung eines mit Schutzgruppen versehenen sternförmigen Polypeptids.

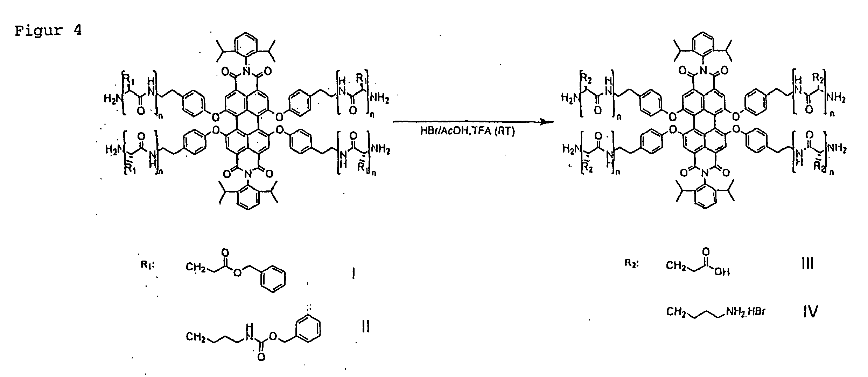

- Figur 4: zeigt die Bildung eines wasserlöslichen sternförmigen Polypeptids, welches auch in wässriger Lösung fluoreszierend ist.

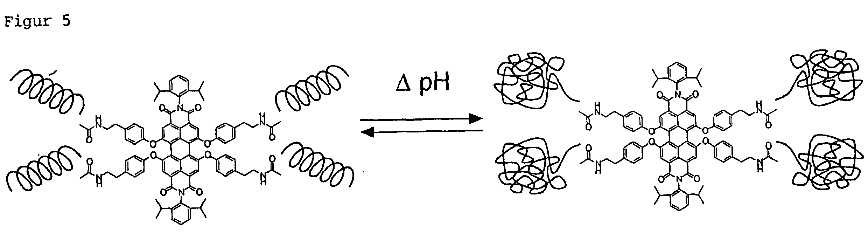

- Figur 5: zeigt die pH-Wertabhängigkeit der Konformation der Polypeptidketten am Farbstoffinitiator.

### Beispiele

Verwendete Abkürzungen:

| | |
|---|---|
| arom. CH(NMR-Spektrum) | aromatisches, ternäres C-Atom |
| aq | wässrig |
| arom. Q(NMR-Spektrum) | aromatisches, quartäres C-Atom |
| ber | berechnet |
| δ | Chemische Verschiebung [ppm] |
| d(NMR-Spektrum) | Dublett |
| DC | Dünnschichtchromatographie |
| DMF | Dimethylformamid |
| DP | Polymerisationsgrad (degree of polymerization) |
| DSC | Differential Scanning Calorimetry |
| FD (Massenspektrum) | Field Desorption |
| GPC | Gelpermeationschromatographie |
| h | Stunden |
| h (NMR-Spektrum) | Heptett |
| λ | Wellenlänge |

| | |
|---|---|
| konz | konzentriert |
| LM | Lösungsmittel |
| m (NMR-Spektrum) | Multiplett |
| M | Molare Masse [g/mol] |
| Mₙ | Zahlenmittel |
| M_{w} | Gewichtsmittel |
| NMP | N-Methyl-2-pyrrolidon |
| NMR | Nuclear Magnetic Resonance |
| ν | Wellenzahl |
| PACL | Poly(tert.-amylcaprolacton) |
| PCL | Polycaprolacton |
| Pd(PPh₃)₄ | Tetrakis-(triphenylphospino)-palladium(0) |
| PL | Polylactid |
| PLLA | Poly(L-lactid) |
| PS | Polystyrol |
| RT | Raumtemperatur |
| s (NMR-Spektrum) | Singulett |
| sh | shoulder |
| TEA | Triethylamin |
| TGA | Thermogravimetrische Analyse |
| THF | Tetrahydrofuran |
| tr (NMR-Spektrum) | Triplett |
| z | Ladungszahl |

### Beispiel 1

### 1. N,N'-Bis(2,6-Diisopropylphenyl)-1,6,7,12-tetrachlor-3,4,9,10-perylentetracarbonsäurediimid

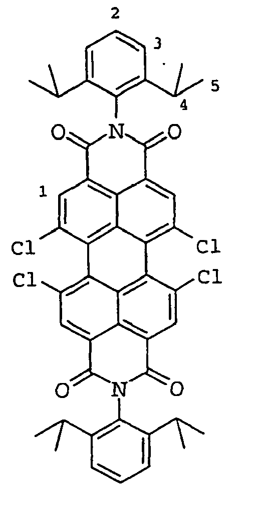

In einem 500-ml-Rundkolben mit Rückflußkühler werden 10 g (0,019 mol) 1,6,7,12-Tetrachlorperylen-3,4,9,10-tetracarbonsäuredianhydrid, 16,67 g (0,094 mol) 2,6-Diisopropylanilin und 250 ml Propionsäure 17 h unter Argon zum Sieden erhitzt, und anschließend, nach Abkühlen auf Raumtemperatur, wird der entstandene Feststoff über eine D4-Glasfritte abfiltriert. Der Rückstand wird portionsweise mit 2 1 Wasser/Methanol (1:3) nachgewaschen und über Nacht im Vakuumtrockenschrank bei 75°C getrocknet.
Ausbeute:
13,61 g (84,4 %) orange-roter Feststoff.
Schmelzpunkt:
>300°C
¹H-NMR-Spektrum (250 MHz, C₂D₂Cl₄, 25°C):
δ (ppm) = 8,11 (s, 4H, H-1), 7,34 (tr, ³J = 7,6 Hz, 2H, H-2), 7,18 (d, ³J = 7,6 Hz, 4H, H-3), 2,63 (h, ³J = 7,1 Hz, 4H, H-4), 1,10 (d, ³J = 7,1 Hz, 24H, H-5)
¹³CNMR-Spektrum (Spinecho-Experiment, 62,5 MHz, C₂D₂Cl₄, 25°C):
δ (ppm) = 162,50 (C=O), 145,82 (arom. q), 135,86 (arom. q), 133,61 (arom. CH), 131,94 (arom. q), 130,25 (arom. q), 130,04 (arom. CH), 129,14 (arom. q), 124,53 (arom. CH), 124,2 (arom. q), 123,47 (arom. q), 29,52 (CH _{isopropyl}), 24, 44 (CH_{3 isopropyl})
FD-Massenspektrum (8 kV):
m/z (u e₀⁻¹) = 846,0 (100%,M⁺) (ber.: 846,16)
IR-Spektrum:
ν = 1701 (ν_{c=o}), 1654 (ν_{c=o}), 1579, 1353, 1279, 1204, 878 cm⁻¹
Elementaranalyse:

| C₄₈H₃₈Cl₄N₂O₄ | C | H | N |
|---|---|---|---|
| Berechnet | 67,93 % | 4,51 % | 3,30 % |
| Gefunden | 67,88 % | 4,45 % | 3,41 % |

### 2. N,N'-Bis(2,6-diisopropylphenyl)-1,6,7,12-tetra-[4-(2-hydroxyethyl)phenoxy]perylen-3,4,9,10-tetracarbonsäurediimid

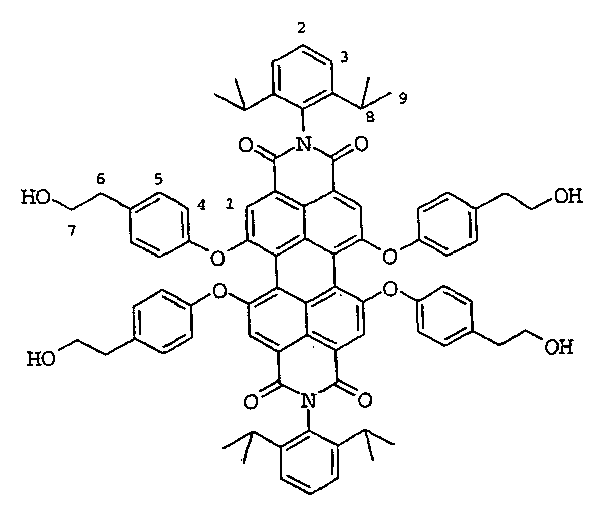

In einem 500-ml-Rundkolben werden 5 g (0,006 mol) N,N'-Bis(2,6-diisopropylphenyl)-1,6,7,12-tetrachlor-3,4,9,10-Perylentetracarbonsäurediimid (1), 8,14 g (0,060 mol) 4-Hydroxyphenethylalkohol und 4,07 g (0,029 mol) K₂CO₃ in 250 ml NMP 12 h unter einer Argonatmosphäre bei 90°C gerührt. Nach Abkühlen auf Raumtemperatur wird das Reaktionsgemisch auf 1 l einer Mischung aus 0,5 Teilen H₂O, 4 Teilen Methanol und 0,2 Teilen konz. HCl gegossen, ca. 2 h gerührt und anschließend über eine D4-Glasfritte filtriert. Der erhaltene Feststoff wird im Vakuum bei 60°C getrocknet und anschließend an Kieselgel mit CH₂Cl₂/Aceton im Verhältnis 1:1 chromatographisch gereinigt.
Ausbeute:
5,56 g (75 %) dunkelroter Feststoff
Schmelzpunkt:
>300°C
¹H-NMR-Spektrum (300 MHz, C₂D₂Cl₄, 25°C):
δ (ppm) = 8,14 (s, 4H, H-1), 7,33 (tr, ³J = 9,2 Hz, 2H, H-2), 7,18 (d, ³J = 9,2 Hz, 4H, H-3), 7,03 (d, ³J = 9,0 Hz, 8H, H-5), 6,89 (d, ³J = 9,1 Hz, 8H, H-4), 3,69 (tr, ³J = 7,2 Hz, 8 H, H-7), 2,70 (tr, ³J = 7,2 Hz, 8H, H-6), 2,60 (h, ³J = 7,6 Hz, 4H, H-8), 1,06 (d, ³J = 7,6 Hz, 24 H, H-9)
¹³C-NMR-Spektrum (Spinecho-Experiment, 75 MHz, C₂D₂Cl₄, 25°C)
δ (ppm) = 163,71 (C=O), 156,04 (arom. q), 154,06 (arom. q), 145,69 (arom. q), 135,30 (arom. q), 133,57 (arom. q), 130,67 (arom. CH), 130,63 (arom. q), 129,68 (arom. CH), 129,65 (arom. CH), 124,20 (arom. CH), 122,87 (arom. q), 121,09 (arom. q), 120,53 (arom. q), 120,29 (arom. CH), 63,53 (CH₂OH), 38,58 (CH₂), 29,43 (CH isopropyl) 24,05 (CH₃ isopropyl)
FD-Massenspektrum:
m/z (u e₀⁻¹) = 1255,4 (100%,M⁺) (ber.: 1255,45)
IR-Spektrum:
ν = 2962, 2868, 1704 (ν_{c=o}) . 1665 (ν_{c=o}), 1585, 1501, 1407, 1285, 1201, 1046, 877, 572 cm⁻¹
UV-Spektrum (CHCl₃):
λₘₐₓ (ε) = 449(15477), 538(28407), 580 nm (49579 1 mol⁻¹ cm⁻¹)
Fluoreszenzspektrum (CHCl₃):
λₘₐₓ = 611 nm
Elementaranalyse:

| C₈₀H₇₄N₂O₁₂ | C | H | N |
|---|---|---|---|
| Berechnet | 62,45 % | 3,78 % | 4,05 % |
| Gefunden | 61,62 % | 3,77 % | 3,94 % |

### 3. N,N'-Bis(2,6-diisopropylphenyl)-1,6,7,12-tetra-[4-(2-(N-butoxycarbonyl)-aminoethyl)phenoxy]perylen-3,4,9, 10-tetracarbonsäurediimid

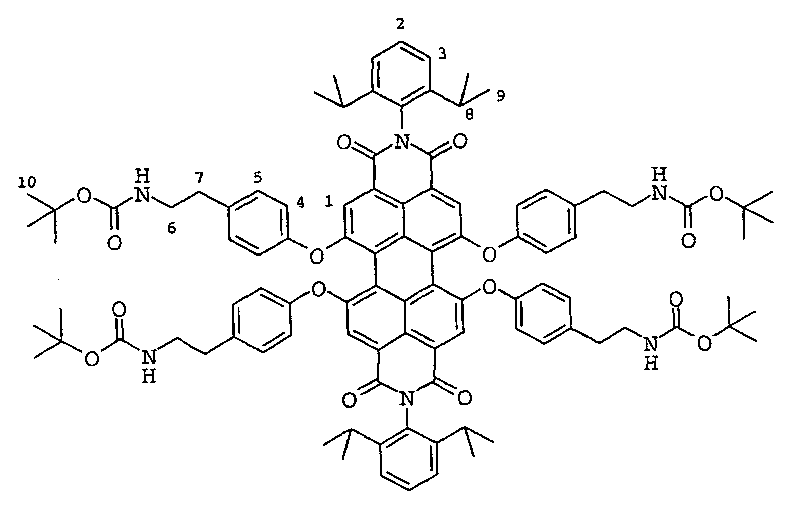

In einem 500-ml-Rundkolben werden 5 g (5,9 mmol) N,N'-Bis(2,6-diisopropylphenyl)-1,6,7,12-tetrachlor-3,4,9,10-perylentetracarbonsäurediimid (1), 13,98 g (0,0589 mol) N-Butoxycarbonyl-(4-hydroxyphenyl)ethylamin und 6,08 g (0,044 mol) K₂CO₃ in 250 ml NMP analog 2. zur Reaktion gebracht. Zur Aufarbeitung wird das Reaktionsgemisch auf 1 1 einer Mischung aus 4 Teilen Methanol und einem Teil 5 %iger wässriger Zitronensäure gegossen, ca. 2 h gerührt und anschließend über eine D4-Glasfritte filtriert. Der erhaltene Feststoff wird im Vakuum bei 60°C getrocknet und anschließend an Kieselgel mit CH₂Cl₂/Aceton im Verhältnis 1:1 chromatographisch gereinigt.
Ausbeute:
7,02 g (72 %) dunkelroter Feststoff
Schmelzpunkt :
241 - 242°C (unter Zersetzung)
¹H-NMR-Spektrum (300 MHz, C₂D₂Cl₄, 25°C):
δ (ppm) = 8,15 (s, 4H, H-1), 7,32 (tr, ³J = 8,2 Hz, 2H, H-2), 7,17 (d, ³J = 8,2 Hz, 4H, H-3), 7,00 (d, ³J = 8,0 Hz, 8H, H-4), 6,86 (d, ³J = 8,0 Hz, 8H, H-5), 4,60 (tr, ³J = 8,9 Hz, 4 H, H-6), 3,2 (s, NH), 2,62 (m, 12H, H-7 u. H-8), 1,35 (s, 36H, H-10), 1,02 (d, ³J =5,9 Hz, 24 H, H-9)
¹³C-NMR-Spektrum (Spinecho, 75 MHz, C₂D₂Cl₄, 25°C):
δ (ppm) = 163, 5 (C=O), 156, 17 (Cq tert.-butyl), 156,04 (arom. q), 154,01 (arom. q), 145,71 (arom. q), 135,77 (arom. q), 133,49 (arom. q), 130,71 (arom. q), 130,40 (arom. CH), 129,62 (arom. CH), 124,17 (arom. CH), 122,89 (arom. CH), 121,04 (arom. q), 120,49 (arom. q), 120,42 (arom. q), 120,25 (arom. CH), 79,44 (Cq _{tert.-butyl}), 42,14 (CH₂NH₂) , 35,78 (CH_{3 tert.-butyl)} , 29,30 (CH _{isopropyl}), 28,76 (CH₂) , 24,39 (CH_{3 isopropyl})
FD-Massenspektrum:
m/z (u e₀⁻¹) = 1650,3 (100%,M⁺) (ber. : 1650,80)
IR-Spektrum:
ν = 3371, 2965, 2930, 2869, 1703 (ν_{c=o}) , 1672 (ν_{c=o)}, 1586, 1501, 1285, 1165, 878, 540 cm⁻¹
UV-Spektrum (CHCl₃) :
λₘₐₓ (ε) = 449 (17984) , 539 (30654) , 579 nm (49747 l mol⁻¹ cm⁻¹)
Fluoreszenzspektrum (CHCl₃):
λₘₐₓ = 611 nm
Elementaranalyse:

| C₁₀₀H₁₁₀N₆O₁₆ | C | H | N |
|---|---|---|---|
| Berechnet | 62,45 % | 3,78 % | 4,05 % |
| Gefunden | 61,62 % | 3,77 % | 3,94 % |

### 4. N,N'-Bis(2,6-diisopropylphenyl)-1,6,7,12-tetra-[4-(2-aminoethyl)phenoxy]perylen-3,4,9,10-tetracarbonsäurediimid

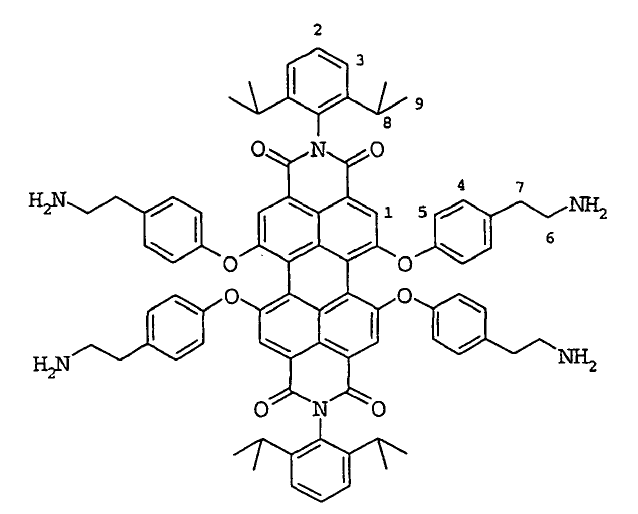

5 g (3,03 mmol) N,N'-Bis(2,6-diisopropylphenyl)-1,6,7,12-tetra-[4-(2-(N-butoxycarbonyl)-aminoethyl)phenoxy]perylen-3,4,9,10-tetracarbonsäurediimid werden in 50 ml CH₂Cl₂ in einem 100-ml-Kolben gelöst und mit einem Eis-Kochsalz-Bad auf 0°C gekühlt. Danach werden langsam 50 ml Trifluoressigsäure über einen Tropftrichter zugetropft, und das Reaktionsgemisch wird 1 h in der Kälte gerührt. Anschließend läßt man noch 1 h bei Raumtemperatur nachrühren und engt am Rotationsverdampfer bis zur Trockene ein. Der Rückstand wird in Dioxan unter Zugabe von wenig 25%iger wässriger Ammoniaklösung gelöst und wieder im Vakuum bis zur Hälfte des Volumens eingeengt. Nach nochmaliger Zugabe von 25 %iger wässriger Ammoniaklösung wird der entstandene Niederschlag über eine D3-Glasfritte abfiltriert, zweimal mit 25 %iger wässriger Ammoniaklösung und zweimal mit Wasser nachgewaschen. Der pulvrige Rückstand wird im Feinvakuum getrocknet.
Ausbeute:
3,72 g (98 %) dunkelroter Feststoff
Schmelzpunkt:
>300 °C
¹H-NMR-Spektrum (500 MHz, CD₂Cl₂, 25°C):
δ (ppm) = 8,18 (s, 4H, H-1), 7,44 (tr, ³J = 8,2 Hz, 2H, H-2), 7,28 (d, ³J = 8,2 Hz, 4k, H-3), 7,12 (d, ³J = 8,8 Hz, 8H, H-4), 6,93 (d, ³J = 8,7 Hz, 8H, H-5), 2,88 (tr, 3J = 7,4 Hz, 8H, H-6), 2,68 (m, 12 H, H-7 u. H-8), 1,41 (d, ³J = 9,1 Hz, NH₂), 1,08 (d, ³J = 6,4 Hz, 24 H, H-9)
¹³C-NMR-Spektrum (Spinecho-Experiment, 125 MHz, CD₂Cl₂, 25°C):
δ (ppm) = 163,7 (C=O), 156,35 (arom. q), 154,05 (arom. q), 146,37 (arom. q), 137,08 (arom. q), 133,59 (arom. q), 130,63 (arom. CH), 129,76 (arom. CH), 124,35 (arom. CH), 123,14 (arom. q), 121,18 (arom. q), 120,66 (arom. q), 120,39 (arom. CH), 120,19 (arom. CH), 43,97 (CH₂NH₂), 39,84 (CH₂), 29,43 (CH _{isopropyl}), 24,05 (CH_{3 isopropyl})
FD-Massenspektrum:
m/z (u e₀⁻¹) = 1251,4 (100%,M⁺) (ber.: 1250,59)
IR-Spektrum:
ν = 3369 (NH₂), 3064, 2962, 2928, 2868, 1704 (ν_{c=o}) , 1670 (ν_{c=o}) , 1586, 1501, 1340, 1284, 1201, 878, 539 cm⁻¹
UV-Spektrum (CHCl₃):
λₘₐₓ = 612 nm
Elementaranalyse:

| C₈₀H₇₈N₆O₈ | C | H | N |
|---|---|---|---|
| Berechnet | 76,78 % | 6,28 % | 6,72 % |
| Gefunden | 76,75 % | 6,31 % | 6,83 % |

### 5. N,N'-Bis(4-brom-2,6-diisopropylphenyl)-1,6,7,12-tetrachlor-3,4,9,10-tetracarbonsäurediimid

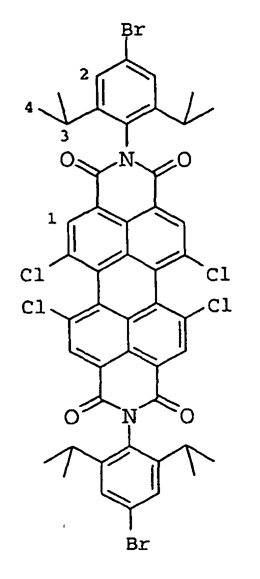

In einem 500-ml-Einhalskolben werden 10 g (0,019 mol) 1,6,7,12-Tetrachlorperylen-3,4,9,10-tetracarbonsäuredianhydrid, 24,16 g (0,094 mol) 4-Brom-2,6-diisopropylanilin und 250 ml Propionsäure 17 h unter Argon zum Rückfluß erhitzt, und anschließend, nach Abkühlen auf Raumtemperatur, wird der entstandene Feststoff über eine D3-Glasfritte abfiltriert. Der Rückstand wird portionsweise mit 1 l Wasser/Methanol (1:3) gewaschen und über Nacht im Vakuumtrockenschrank bei 75°C getrocknet.
Ausbeute:
15,4 g (80,53 %) orange-roter Feststoff
Schmelzpunkt:
>300°C
¹H-NMR-Spektrum (250 MHz, C₂D₂Cl₄, 25°C):
δ(ppm) = 8,68 (s, 4H, H-1), 7,28 (s, 4H, H-2), 2,61 (h, ³J = 6,4 Hz, 4H, H-3), 1,18 (d, ³J = 4,6 Hz, 24 H, H-4)
¹³C-NMR-Spektrum (Spinecho-Experiment, 62,5 MHz, THF-d₈, 25°C):
δ (ppm) = 162,89 (C=O), 149,59 (arom. q), 136,17 (arom q), 133,80 (arom. CH), 132,72 (arom. q), 130,80 (arom. q), 129,60 (arom. q), 128,27 (arom. CH), 125,34 (arom. q), 124,63 (arom. q), 110,21 (arom. q), 30,09 (CH _{isopropyl}), 24,03 (CH_{3 isopropyl})
FD-Massenspektrum:
m/z (u e₀⁻¹) = 1001,4 (100%,M⁺) (ber.: 1001,98)
UV-Spektrum (CHCl₃):
λₘₐₓ (ε) = 427 (11078), 488 (33922), 522 nm (49517 1 mol⁻¹ cm⁻¹)
IR-Spektrum:
ν= 1704 (ν_{c=o}), 1663 (ν_{c=o}), 1585, 1401, 1356, 1301, 1201, 852 cm⁻¹
Elementaranalyse:

| C₄₉H₃₆Br₂Cl₄N₂O₄ | C | H | N |
|---|---|---|---|
| Berechnet | 57,28 % | 3,61 % | 2,78 |
| Gefunden | 57,41 % | 3,63 % | 2,75 |

### 6. N,N'-Bis(4-brom-2,6-diisopropylphenyl)-1,6,7,12-tetra-[4-(2-hydroxyethyl)phenoxy]perylen-3,4,9,10-tetracarbonsäurediimid

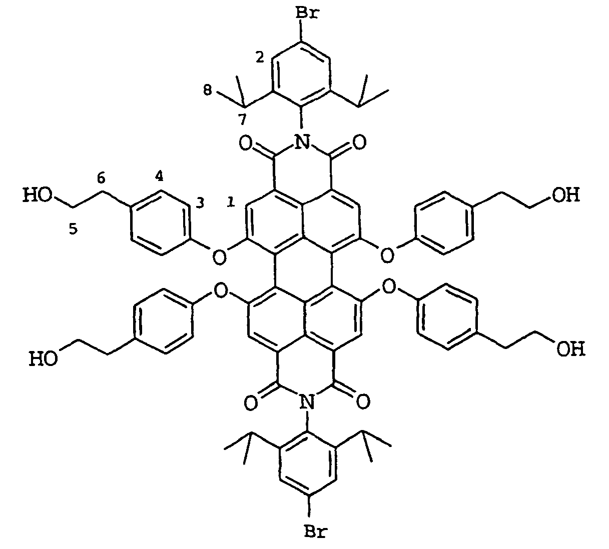

5 g (0,005 mol) N,N'-Bis(4-brom-2,6 diisopropylphenyl)-1,6,7,12-tetrachlor-3,4,9,10-perylentetracarbonsäurediimid (5), 6,91 g (0,050 mol) 4-Hydroxyphenylethanol und 3,45 g (0,025 mol) K₂CO₃ werden in 250 ml NMP 12 h unter Argon bei 90°C gerührt. Nach Kontrolle mittels DC (Laufmittel: CH₂Cl₂/Aceton 1:1) wird das Reaktionsgemisch auf 1 1 einer Mischung aus 0,5 Teilen Wasser, 4 Teilen Methanol und 0,2 Teilen konz. Salzsäure gegossen, 2 h gerührt und anschließend über eine D4-Glasfritte filtriert. Der erhaltene Feststoff wird im Vakuum bei 60°C getrocknet und anschließend an Kieselgel mit CH₂Cl₂/Aceton im Verhältnis 1:1 chromatographisch gereinigt.
Ausbeute:
4,38 g (62 %) dunkelroter, pulvriger Feststoff
Schmelzpunkt:
>300°C
¹H-NMR-Spektrum (250 MHz, C₂D₂Cl₄, 25°C)
δ (ppm) = 8,13 (s, 4H, H-1), 7,28 (s, 4H, H-2), 7,02 (d, ³J = 7,7 Hz, 8 H, H-3), 6,87 (d, ³J = 7,9 Hz, 8H, H-4), 3,69 (m, 12H, H-5 u. OH), 2,70 (tr, ³J = 7,8 Hz, 8H, H-6), 2,56 (h, ³J = 7,1 Hz, 4 H, H-7), 1,01 (d, ³J = 7,1, 24H, H-8)
¹³C-NMR-Spektrum (Spinecho-Experiment, 62,5 MHz, C₂D₂Cl₄, 25°C)
δ (ppm) = 163,37 (C=O), 156,22 (arom. q), 153,95 (arom. q), 148,38 (arom. q), 135,40 (arom. q), 133,55 (arom. q), 130,72 (arom. CH), 130,00 (arom. q), 127,66 (arom. CH), 123,88 (arom. q), 122,60 (arom. q), 121,21 (arom. q), 120,507 (arom. q), 120,38 (arom. CH), 120,26 (arom. CH), 63,52 (CH₂OH), 38,55 (CH₂), 29,44, 25,94 (CH _{isopropyl}), 24,22 (CH_{3 isopropyl})
FD-Massenspektrum:
m/z (u e₀⁻¹) = 1410,4 (100%, M⁺) (ber.: 1410,35)
IR-Spektrum:
ν = 1704 (ν_{c=o}) , 1684 (ν_{c=o}), 1672, 1586, 1500, 1408, 1341, 1311, 1284, 1201, 1051, 878 cm⁻¹
UV-Spektrum (CHCl₃):
λₘₐₓ (ε) = 450 (15561), 541 (29261), 581 nm (49831 1 mol⁻¹ cm⁻¹)
Elementaranalyse:

| C₈₀H₇₂Br₂N₂O₁₂ | C | H | N |
|---|---|---|---|
| Berechnet | 67,99 % | 5,14 % | 1,98 % |
| Gefunden | 67,83 % | 5,30 % | 1,79 % |

### 7. N,N'-Bis[4-(butin-1-ol)-2,6-diisopropylphenyl]-1,6,7,12-tetra-[4-(4-hydroxyethyl)phenoxy]perylen-3,4,9,10-tetracarbonsäurediimid

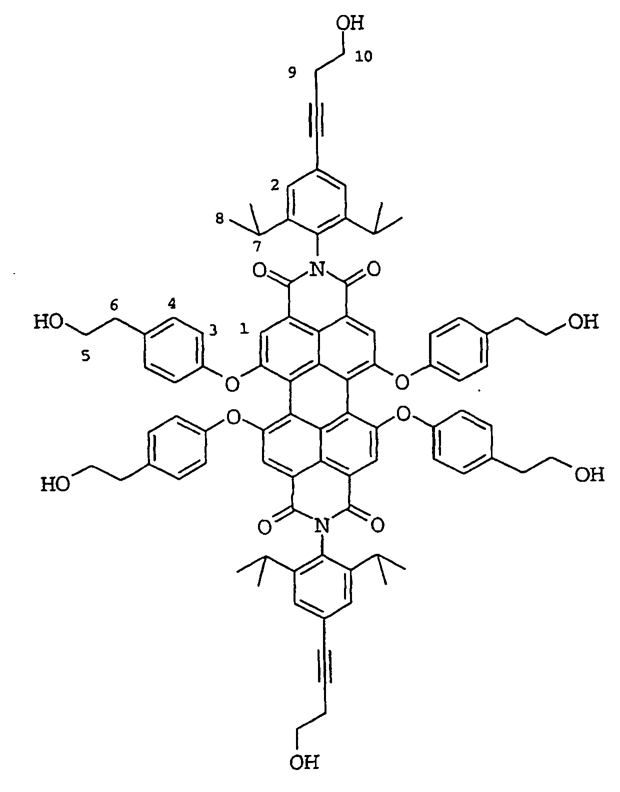

In einer Argonatmosphäre mischt man je 44 ml THF und Piperidin in einem 500-ml-Schlenkkolben. Im Argongegenstrom gibt man 3,00 g (2,12 mmol) N,N'-Bis(4-brom-2,6-diisopropylphenyl)-1,6,7,12-tetra-[4-(2-hydroxyethyl)phenoxy]perylen-3,4,9,10-tetracarbonsäurediimid (6), 0,195 g (0,169 mmol) Pd(PPh₃)₄ und 0,040 g (0,212 mmol) Kupfer(I)iodid hinzu. Der Kolben wird mit einem Septum verschlossen, bevor man mit einer Spritze 0,594 g (8,48 mmol) Butinol zusetzt und das Reaktionsgemisch auf 50°C erwärmt. Nach 24 h wird die Temperatur auf 80°C erhöht. Nach weiteren 24 h Reaktionszeit wird auf eine Mischung aus Eis und konz. Salzsäure (3:1) gegossen und ca. 1 h gerührt. Das ausgefallene Produkt wird über eine D3-Glasfritte abgesaugt, im Feinvakuum getrocknet und mit CH₂Cl₂/Ethanol (90:10) an Kieselgel chromatographiert.
Ausbeute:
2,3 g (78 %) dunkelroter, pulvriger Feststoff
Schmelzpunkt:
>300°C
¹H-NMR-Spektrum (250 MHz, C₂D₂Cl₄, 25°C):
δ (ppm) = 8,15 (s, 4H, H-1), 7,24 (s, 4H, H-2), 7,04 (d, ³J = 7,8 Hz, 8H, H-3), 6,88 (d, ³J = 7,9 Hz, 8H, H-4), 3,74 (m, 12H, H-5 u. H-10), 2,67 (m, 16H, H-6, H-7, H-9), 1,02 (d, ³J = 7,1 Hz, 24H, H-8)
¹³C-NMR-Spektrum (Spinecho-Experiment, 75 MHz, C₂D₂Cl₄, 25°C):
δ (ppm) = 163,4 (C=O), 156,16 (arom. q), 154,04 (arom q), 146,15 (arom. q), 135,29 (arom. q), 133,55 (arom. q), 130,72 (arom. CH), 127,84 (arom. q), 125,02 (arom. CH), 124,40 (arom. q), 122,74 (arom. q), 121,19 (arom. q), 120,55 (arom. q), 120,30 (arom. CH), 120,25 (arom. CH), 86,95 (C≡C), 82,98 (C≡C), 74,46 (CH₂), 63,52 (CH₂)_{'} 61, 39 (CH₂) , 38, 53 (CH₂) , 29,25 (CH _{isopropyl}), 24,26 (CH_{3 isopropyl})
FD-Massenspektrum:
m/z (u e₀⁻¹) = 1391,5 (100%, M⁺) (ber.: 1390,58)
IR-Spektrum:
ν = 2961, 1726 (ν_{c=o}), 1689, 1582, 1502, 1468, 1334, 1295, 1200, 1045, 878 cm⁻¹
UV-Spektrum (CHCl₃):
λₘₐₓ (ε) - 451 (15822), 539 (28988), 580 nm (49693 1 mol⁻¹ cm⁻¹)
Fluoreszenzspektrum (CHCl₃):
λₘₐₓ = 610 nm

| C₈₈H₈₂N₂N₂O₁₄ | C | H | N |
|---|---|---|---|
| Berechnet | 75,95 % | 5,94 % | 2,01 % |
| Gefunden | 75,17 % | 6,08 % | 1,89 % |

### 8. N,N'-Bis-(2,6-diisopropylphenyl)-1,6,7,12-tetra-[4-(ethyl-2-(2-bromisobuttersäureamid)phenoxy]perylen-3,4,9,10-tetracarbonsäurediimid

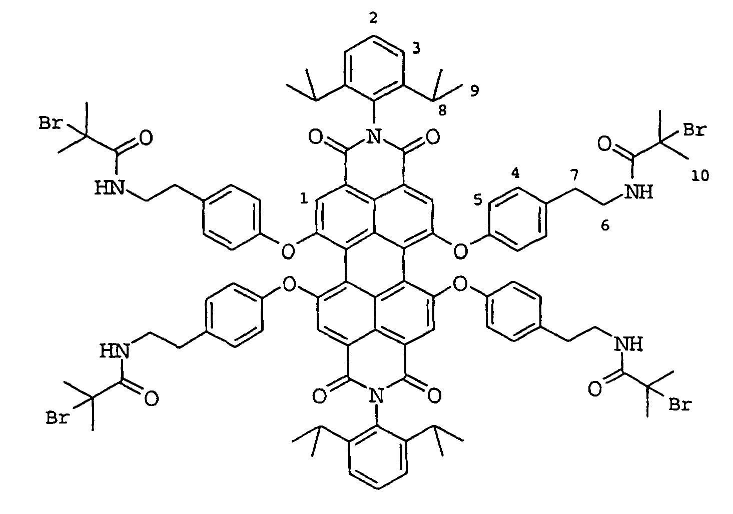

3,12 g (0,0025 mol) N,N'-Bis(2,6-diisopropylphenyl)-1,6,7,12-tetra-[4-(2-aminoethyl)phenoxy]perylen-3,4,9,10-tetracarbonsäurediimid (4) und 0,3 g (0,003 mol) Triethylamin werden in einem 500-ml-Dreihalskolben mit Innenthermometer in 200 ml trockenem CH₂Cl₂ gelöst, unter Argon gesetzt und im Eis-Kochsalz-Bad auf 0°C gekühlt. Anschließend werden 7,4 g (0,032 mol) 2-Bromisobuttersäurebromid über einen Tropftrichter langsam zugetropft, so daß die Temperatur nicht ansteigt. Nach 2 h Reaktionszeit (Kontrolle durch DC) wird der entstandene Niederschlag (Triethylammoniumbromid) abfiltriert, und das Filtrat wird zweimal mit verdünnter wässriger Salzsäure und zweimal mit verdünnter wässriger Natriumcarbonatlösung gewaschen. Die organische Phase wird abgetrennt, über Natriumsulfat getrocknet, und das Lösungsmittel wird im Vakuum entfernt. Das Produkt wird an Kieselgel mit CH₂Cl₂ als Eluent chromatographiert.
Ausbeute:
4,2 g (91,2 %) dunkelroter Feststoff
Schmelzpunkt:
>300°C
¹H-NMR-Spektrum (250 MHz, C₂D₂Cl₄, 25°C) :
δ (ppm) = 8,78 (s, 4H, H-1), 8,00 (tr, ³J = 8,2 Hz, 2H, H-2), 7,84 (d, ³J=8,2 Hz, 4H, H-3), 7,71 (d, ³J = 6,8 Hz, 8H, H-4), 7,54 (d, ³J = 6,8 Hz, 8H, H-5), 7,36 (tr, ³J = 8,0 Hz, 4H, NH), 4,03 (s, 8H, H-6), 3,38 (tr, ³J = 7,9 Hz, 8H, H-7), 3,26 (h, ³J = 7,8 Hz, 4H, H-8), 2,51 (s, 24H, H-10), 1,69 (d, ³J = 8,0 Hz, 24H, H-9)
¹³C-NMR-Spektrum (Spinecho-Experiment, 62,5 MHz, C₂D₂Cl₄, 25°C) :
δ (ppm) = 172,13 (C=O_{amid}), 163,49 (C=O_{imid}) , 156,25 (arom. q), 154,16 (arom. q), 145,70 (arom. q), 135,41 (arom. q), 133,47 (arom. q), 130,67 (arom. q), 130,57 (arom. CH), 129,64 (arom. CH), 124,21 (arom. CH), 122,89 (arom. q), 120,97 (arom. q), 120,33 (arom. CH), 120,45 (arom. CH), 63,05 (Cq _{isobrombutyrat}) , 42,00 (CH₂), 35, 01 (CH₂), 32,72 (CH_{3 isobrombutyrat}), 29,28 (CH _{isopropyl}), 24,43 (CH_{3 isopropyl})
FD-Massenspektrum:
m/z (u eₒ⁻¹) = 1842,8 (100%,M⁺) (ber.: 1842,40)
IR-Spektrum:
ν = 3031, 2963, 2868, 1704 (ν_{c=o}), 1665 (ν_{c=o}), 1585, 1503, 1407, 1284, 1202, 1056, 1015, 878 cm⁻¹
UV-Spektrum (CHCl₃):
λₘₐₓ (ε) = 447 (16361), 537 (28035), 577 nm (46036 1 mol⁻¹ cm^{.1})
Fluoreszenzspektrum (CHCl₃):
λₘₐₓ = 614 nm
Elementaranalyse:

| C₉₆H₉₈Br₄N₆O₁₂ | C | H | N |
|---|---|---|---|
| Berechnet | 62,41 % | 5,35 % | 4,55 % |
| Gefunden | 61,92 % | 5,51 % | 4,65 % |

### 9. N,N'-His(2,6-diisopropylphenyl)-1,6,7,12-tetra-[4-(ethyl-2-(2-bromisobuttersäureester)phenoxy]perylen-3,4,9,10-tetracarbonsäurediimid

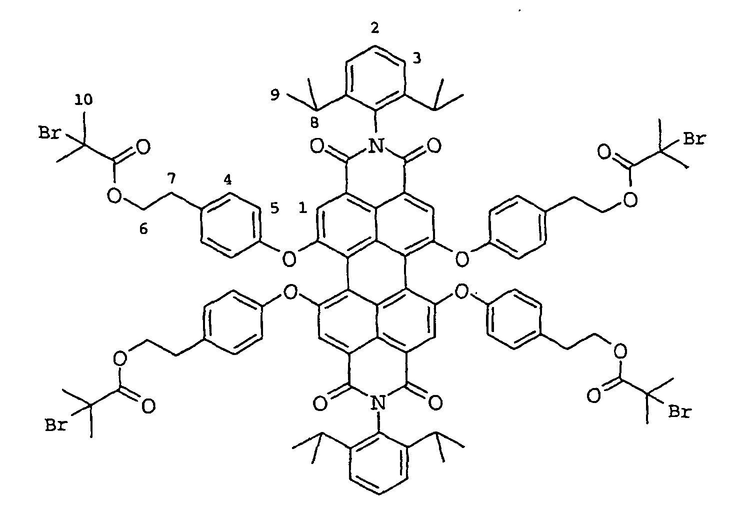

5 g (3,98 mmol) N,N'-Bis(2,6-diisopropylphenyl)-1,6,7,12-tetra-[4-(4-hydroxyethyl)phenoxy]perylen-3, 4,9, 10-tetracarbonsäurediimid (2) werden mit 7,32 g (0,031 mol) 2-Bromisobuttersäurebromid unter Zusatz von 0,3 g (0,003 mol) Triethylamin analog 8. umgesetzt.
Ausbeute:
6,8 g (93 %) dunkelroter Feststoff
Schmelzpunkt:
>300°C
¹H-NMR-Spektrum (250 MHz, C₂D₂Cl₄, 25°C):
δ (ppm) = 8,78 (s, 4H, H-1), 8,00 (tr, ³J = 8,2 Hz, 2H, H-2), 7,84 (d, ³J = 8,2 Hz, 4H, H-3), 7,71 (d, ³J = 6,8 Hz, 8H, H-4), 7,54 (d, ³J = 6,8 Hz, 8H, H-5), 4,03 (tr, ³J = 7,9, 8H, H-6), 3,38 (tr, ³J = 7,9 Hz, 8H, H-7), 3,26 (h, ³J = 7,9 Hz, 4H, H-8), 2,51 (s, 24H, H-10), 1,69 (d, ³J = 7,9 Hz, 24H, H-9)
¹³C-NMR-Spektrum (Spinecho-Experiment, 125 MHz, C₂D₂Cl₄, 60°C):
δ (ppm) = 172,97, 164,33, 157,09, 155,01, 146,54, 136,25, 134,25, 123,74, 121,82, 63,89, 42,84, 35,86
FD-Massenspektrum:
m/z (u e₀⁻¹) = 1846,8 (100%,M⁺) (ber. : 1846,33)
UV-Spektrum (CHCl₃):
λₘₐₓ (ε) = 448 (20187), 537 (35699), 577 nm (58861 1 mol⁻¹ cm⁻¹)
Fluoreszenzspektrum (CHCl₃):
λₘₐₓ = 614 nm
IR-Spektrum:
ν= 2949, 1741 (ν_{c=o}). 1666 (ν_{c=o}), 1591 (ν_{c=o}). 1499, 1356, 1336, 1264, 1114, 820, 737 cm⁻¹
Elementaranalyse:

| C₉₆H₉₄Br₄O₁₆ | C | H | N |
|---|---|---|---|
| Berechnet | 62,98 % | 5,12 % | 1,51 % |
| Gefunden | 62,92 % | 5,16 % | 1,53 % |

### 10. N,N'-Bis(2,6-diisopropylphenyl)-1,7-di-[4-(2-(N-butoxycarbonyl)aminoethyl)phenoxy]perylen-3,4,9,10-tetracarbonsäurediimid

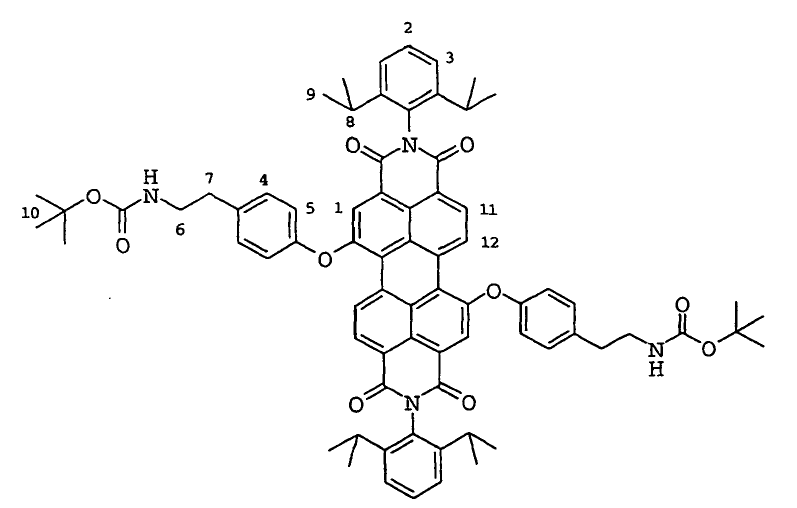

Analog zu 3. werden 6 g (6,9 mmol) N,N'-Bis(2,6-diisopropylphenyl)-1,7-dibrom-3,4,9,10-perylentetracarbonsäurediimid, 5,545 g (23,4 mmol) BOC-geschütztes Tyramin und 2,42 g (17,5 mmol) K₂CO₃ in 250 ml NMP zur Reaktion gebracht.
Ausbeute:
4,5 g (55,2 %) oranger Feststoff mit deutlicher Feststofffluoreszenz
Schmelzpunkt:
284 - 285°C (Zersetzung)
¹H-NMR-Spektrum (250 MHz, C₂D₂Cl₄, 25°C):
δ (ppm) = 9,65 (d, ³J = 7,9 Hz, 2H, H-11), 8,66 (d, ³J = 7,9 Hz, 2H, H-12), 8,38 (s, 2H, H-1), 7,48 (tr, ³J = 6,9 Hz, 2H, H-2), 7,33 (d, ³J = 8,2 Hz, 4H, H-3), 7,32 (d, ³J = 8,2 Hz, 4H, H-4), 7,15 (d, ³J = 6,9 Hz, 4H, H-5), 3,31 (tr, ³J = 9,3 Hz, 4H, H-6), 2,75 (tr, ³J = 9,3 Hz, 4H, H-7), 2,60 (h, ³J = 8,2 Hz, 4H, H-8), 1,70 (s, NH), 1,34 (s, 18H, H-9), 1,05 (tr, ³J = 8,2 Hz, 24H, H-10)
¹³C-NMR-Spektrum (Spinecho-Experiment, 75 MHz, C₂D₂Cl₄, 25°C) :
δ (ppm) = 163,62 (C=O), 163,22 (C=O), 156,07 (C=O), 155,73 (arom. q), 153,62 (arom. q), 145,75 (arom. q), 136,59 (arom. q), 134,02 (arom. q), 131,20 (arom. CH), 130,99 (arom. CH), 130,73 (arom. q), 129,94 (arom. q), 129,73 (arom. CH), 129,29 (arom. CH), 125,93 (arom. q), 124,40 (arom. q), 124,30 (arom. CH), 124,17 (arom. CH), 124,10 (arom. q), 122,48 (arom. q), 120,06 (arom. CH), 79,51 (C_{q tert.-butyl}) 42,07 (CH₂NH₂), 35,97 (CH_{3 tert. -butyl}), 29,40 (CH _{isopropyl}), 28, 73 (CH₂), 24, 39 (CH_{3 isopropyl})
FD-Massenspektrum:
m/z (u e₀⁻¹) = 1180,5 (100%,M⁺) (ber.: 1181,42)
UV-Spektrum (CHCl₃):
λₘₐₓ (ε) = 401 (9375), 509 (34332), 541 nm (45726 l mol⁻¹ cm⁻¹)
IR-Spektrum:
ν = 3353, 3059, 2960, 2923, 2859, 1701 (ν_{c=o}), 1659 (ν_{c=o}) , 1586, 1353, 1298, 1270, 872, 815 cm⁻¹
Fluoreszenzspektrum (CHCl₃):
λₘₐₓ = 578, 617 (sh) nm
Elementaranalyse:

| C₇₄H₇₆N₄O₁₀ | C | H | N |
|---|---|---|---|
| Berechnet | 75,23 % | 6,48 % | 4,74 % |
| Gefunden | 75,14 % | 6,51 % | 4,71 % |

### 11. N,N'-Bis(2,6-diisopropylphenyl)-1,7-di-[4-(2-aminoethyl)-phenoxy]perylen-3,4,9,10-tetracarbonsäurediimid

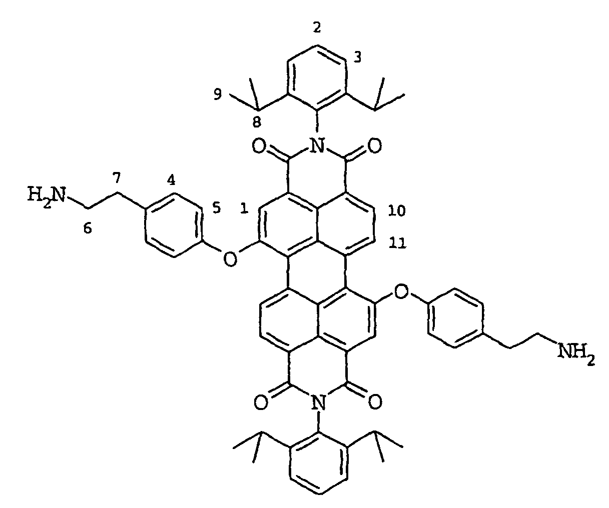

Analog zu 4. werden 5 g (4,2 mmol) N,N'-Bis(2,6-diisopropylphenyl)-1,7-di-[4-(2-(N-butoxycarbonyl)aminoethyl)phenoxy]perylen-3,4,9,10-tetracarbonsäurediimid mit 50 ml Trifluoressigsäure in 50 ml CH₂Cl₂ zur Reaktion gebracht.
Ausbeute:
4,03 g (97 %) oranger Feststoff mit deutlicher Feststofffluoreszenz
Schmelzpunkt:
291 - 293°C
¹H-NMR-Spektrum (250 MHz, C₂D₂Cl₄, 25°C):
δ (ppm) = 9,64 (d,³J = 8,4 Hz, 2H, H-10), 8,66 (d, ³J = 8,4 Hz, 2H, H-11), 8,32 (s, 2H, H-1), 7,48 (tr, ³J = 7,6 Hz, 2H, H-2), 7,34 (d, ³J = 8 Hz, 2H, H-3), 7,32 (d, ³J = 8 Hz, 4H, H-4), 7,15 (d, ³J = 8 Hz, 4H, H-5), 2,97 (tr, ³J = 6,9 Hz, 4H, H-6), 2,77 (tr, ³J = 6,9 Hz, 4H, H-7), 2,71 (h, ³J = 7,4 Hz, 4H, H-8), 1,11 (s, 24H, H-9)
¹³C-NMR-Spektrum (Spinecho-Experiment, 75 MHz, C₂D₂Cl₄, 25°C):
δ (ppm) = 163,62 (C=O), 163,22 (C=O), 155,73 (arom. q), 153,62 (arom. q), 145,75 (arom. q), 136,59 (arom. q), 134,02 (arom. q), 131,20 (arom. CH), 130,99 (arom. CH), 130, 73 (arom. q), 129,94 (arom. q), 129,73 (arom. CH), 129,29 (arom. CH), 125,93 (arom. q), 124,40 (arom. q), 124,30 (arom. CH), 124,17 (arom. CH), 124,10 (arom. q), 122,48 (arom. q), 120,06 (arom. CH), 43,46 (CH₂NH₂), 38,68 (CH₂), 29,40 (CH _{isopropyl}) , 24,39 (CH_{3 isopropyl})
FD-Massenspektrum:
m/z (u eₒ⁻¹) = 981,0 (100%,M⁺) (ber.: 981,18)
UV-Spektrum (CHCl₃):
λₘₐₓ(ε) = 402 (8665), 511 (31350), 543 nm (43466 1 mol⁻¹ cm⁻¹)
IR-Spektrum:
ν = 3353, 2961, 2923, 2867, 1707 (ν_{c=o}), 1665 (ν_{c=o}), 1593, 1502, 1405, 1335, 1260, 1197, 812 cm⁻¹
Fluoreszenzspektrum (CHCl₃):
λₘₐₓ = 578, 617 (sh) nm
Elementaranalyse:

| C₆₄H₆₀N₄O₆ | C | H | N |
|---|---|---|---|
| Berechnet | 78,34 % | 6,16 % | 5,71 % |
| Gefunden | 78,94 % | 6,21 % | 5,68 % |

### 12. N,N'-Bis(2,6-diisopropylphenyl)-1,7-di-[4-(2-hydroxyethyl)phenoxy]perylen-3,4,9,10-tetracarbonsäurediimid

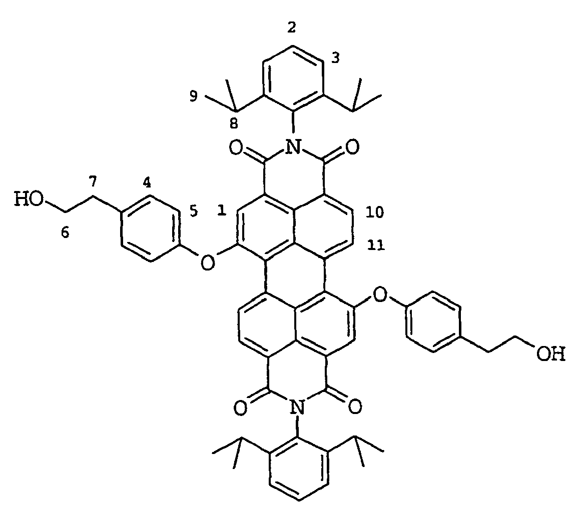

Analog zu 2. werden 5 g (5,7 mmol) N,N'-Bis(2,6-diisopropylphenyl)-1,7-dibromperylen-3,4,9,10-tetracarbonsäurediimid mit 3,18 g (0,023 mol) 4-Hydroxyphenylalkohol zur Reaktion gebracht.
Ausbeute:
3,42 g (61 %) oranger Feststoff mit deutlicher Feststofffluoreszenz
Schmelzpunkt:
291 - 293°C
¹H-NMR-Spektrum (250 MHz, C₂D₂Cl₄, 25°C):
δ (ppm) = 9,54 (d, ³J = 8,2 Hz, 2H, H-1), 8,62 (d, ³J = 8,2 Hz, 2H, H-10), 8,2 (s, 2H, H-11), 7,38 (tr, ³J = 7,9 Hz, 2H, H-2), 7,32 (d, ³J = 8 Hz, 2H, H-3), 7,29 (d, ³J = 8,4 Hz, 4H, H-4), 7,13 (d, ³J = 8,4 Hz, 4H, H-5), 3,81 (tr, ³J = 8,4 Hz, 4H, H-6), 2,82 (tr, ³J = 6,9 Hz, 4H, H-7), 2,61 (h, ³J = 6,9 Hz, 4H, H-8), 1,05 (s, 24H, H-9)
¹³C-NMR-Spektrum (Spinecho-Experiment, 75 MHz, C₂D₂Cl₄, 25°C):
δ (ppm) = 163,66 (C=O), 163,27 (C=O), 155,79 (arom. q), 153,56 (arom. q), 145,75 (arom. q), 136,25 (arom. q), 134,05 (arom. q), 131,45 (arom. CH), 131,01 (arom. CH), 130,70 (arom. q), 129,95 (arom. q), 129,76 (arom. CH), 129,31 (arom. CH), 125,91 (arom. q), 124,38 (arom. CH), 124,31 (arom. CH), 124,05 (arom. q), 122,45 (arom. q), 120,12 (arom. CH), 63,70 (CH₂OH), 38,74 (CH₂), 29, 39 (CH _{isopropyl}), 24,37 (CH_{3 isopropyl})
FD-Massenspektrum:
m/z (u eₒ⁻¹) = 981,0 (100%,M⁺) (ber.: 981,18)
UV-Spektrum (CHCl₃):
λₘₐₓ (ε) = 401 (10578), 544 nm (49482 1 mol⁻¹ cm⁻¹)
IR-Spektrum:
ν = 2961, 2923, 2868, 1705 (ν_{c=o}), 1664 (ν_{c=o}), 1593, 1502, 1406, 1336, 1260, 1197, 1055, 812 cm⁻¹
Fluoreszenzspektrum (CHCl₃):
λₘₐₓ = 579, 615 (sh) nm
Elementaranalyse:

| C₆₄H₅₈N₂O₈ | C | H | N |
|---|---|---|---|
| Berechnet | 78,19 % | 5,95 % | 2,85 % |
| Gefunden | 78,13 % | 5,97 % | 2,82 % |

### 13. tert.-Amylcaprolacton

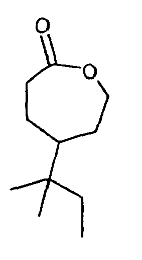

In einem 2-1-Kolben werden 1 l CH₂Cl₂, 10,5 g (0,0715 mol), Trifluormethansulfonsäure als Katalysator und 212,6 g (60 %ig = 1,45 mol) m-Chlorperoxybenzoesäure vorgelegt. Das Gemisch wird im Kältebad auf 0°C gekühlt, und 120 g (0,7 mol) 4-tert.-Amylcyclohexanon werden unter intensivem Rühren rasch zugetropft, wobei die Temperatur nicht über 20°C steigen sollte. Nach beendeter Zugabe wird noch 30 Minuten ohne Kältebad weiter gerührt. Anschließend filtriert man den festen Rückstand (4-Chlorbenzoesäure) über eine Glasfritte ab. Das Filtrat wird zunächst mit 1 l 5 % iger wässriger Natriumhydrogencarbonat- und dann mit gesättigter, wässriger Natriumchloridlösung gewaschen. Das Dichlormethan wird am Rotationsverdampfer abdestilliert, und der gelbliche, ölige Rückstand wird im Feinvakuum getrocknet. Das Produkt wird zur rigorosen Entfernung von Wasserspuren über Nacht bei 100°C mit Calciumhydrid gerührt und anschließend über eine 25 cm Vigreuxkolonne destilliert.
Ausbeute:
125,1 g (97 %) (nach Destillation 93,8 g (86 %)) weißer Feststoff
Schmelzpunkt:
42 - 45°C
Siedepunkt:
110°C (2*10⁻³ mbar)
¹H-NMR-Spektrum (500 MHz, C₂D₂Cl₄, 353 K):
δ (ppm) = 4,08 (m, 1H), 3,94 (m, 1H), 2,40 (m, 2H), 1,80 (m, 1H), 1,73 (m, 1H), 1,25 (m, 2H), 1,1 (m, 3H), 0,65 (m, 9H)
¹³C-NMR-Spektrum (500 MHz, C₂D₂Cl₄, 353 K):
δ (ppm) = 175,19, 68,13, 47,7, 34,88, 33,28, 32,12, 29,87, 23,89, 23,85, 23,23, 7,69
Elementaranalyse:

| C₁₁H₂₀O₂ | C | H |
|---|---|---|
| Berechnet | 71,70 % | 10,94 % |
| Gefunden | 71,71 % | 10,97 % |

### 14. Polymerisationen

Die UV/VIS- und Fluoreszenzspektren der Polymere gleichen jeweils denen der entsprechenden Farbstoffe mit einer Abweichung von +/-2 nm. Es ist zu jeder Polymerart jeweils ein ¹H-NMR Spektrum angegeben. Die Spektren der jeweils analogen Polymere unterscheiden sich lediglich durch die Integrationszahlen, die sich direkt in den ¹H-NMR DP Angaben widerspiegeln. Die Ausbeuten der Polymerisationen liegen durchweg zwischen 95 und 98 %.

### 14.1. Allgemeine Arbeitsvorschrift für ringöffnende lebende Polymerisation von Polylactiden

### Beispielvorschrift:

225 mg (0,20 mmol) des Initiator-Farbstoffmoleküls und 1,44 g (0,02 mol) L,L-Dilactid werden in einer Glovebox in ein Schlenkrohr eingewogen. Das Schlenkrohr wird mit einem Septum verschlossen, aus der Glovebox ausgeschleust und unter Argon im Ölbad auf 90°C erwärmt. Wenn alles Monomer geschmolzen ist, werden 6,4 mg (0,016 mmol) Zinn(II)-(2-ethyl)hexanoat in Form einer 1 %igen Lösung in wasserfreiem Toluol als Katalysator zugespritzt. Die Menge an Katalysator entspricht 1/50 der molaren Menge der OH-Gruppen. Die Temperatur wird auf 110°C erhöht, und die Mischung wird 48 h gerührt. Das dann feste Polymer wird in CH₂Cl₂ gelöst und durch Eintropfen unter Rühren in Methanol ausgefällt, wobei man das Polymer 1 a als rotes Pulver in quantitativer Ausbeute erhält, das anschließend im Feinvakuum getrocknet wird.

1 b, 1 c und 2 a-c werden analog erhalten, wobei jedoch die in der Tabelle angebenen Mengen von Initiator und Monomer eingesetzt werden.

¹H-NMR-Spektrum des 4Arm Sternpolymers 1 a (300 MHz, C₂D₂Cl₄, 25 °C) :
δ (ppm) = 8,08 (s, 4H), 7,32 (tr, 2H), 7,16 (d, 4H), 7,03 (d, 8H), 6,85 (d, 8H), 5,08 (m, 91H), 4,23 (m, 12H), 2,82 (s, 8H), 2,58 (tr, 6H), 1,49 (d, 254H), 1,00 (s, 24H)

### Beispiel ¹H-NMR-Spektrum für 6Arm Sternpolymere:

¹H-NMR-Spektrum der Verbindung 2 a (250 MHz, C₂D₂Cl₄, 25°C) :
δ (ppm) = 8, 10 (s, 4H), 7,20 (s, 4H), 7,04 (d, 8H), 6,84 (d, 8H), 5,10 (q, 143,8 H), 4,25 (m, 18H), 2,86 (d, 8H), 2,75 (h, 4H), 2,61 (tr, 4H), 1,50 (d, 447H), 1,00 (s, 24 H)

### 14.1.1. PLLA 1 a-c

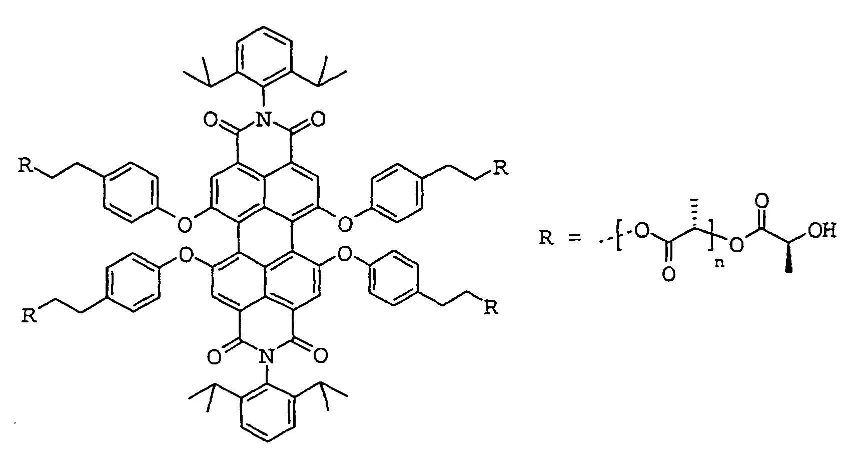

**Tabelle 1:**

| Nr. | DP, angestrebt | Initiator | Monomer | Katalysator |
|---|---|---|---|---|
| 1 a | 25 | 225 mg | 1,44 g | 6,4 mg |
| | | 0,20 mmol | 0,02 mol | 0,016 mmol |
| 1 b | 50 | 225 mg | 2,88 g | 6,4 mg |
| | | 0,20 mmol | 0,04 mol | 0,016 mmol |
| 1 c | 100 | 112,5 mg | 2,88 g | 3,2 mg |
| | | 0,10 mmol | 0,04 mol | 0,008 mmol |

Charakterisierung:

**Tabelle 2: Charakterisierung und thermische Eigenschaften der hergestellten PLLA 4Arm Sterne**

| Nr | DP Ziel | DP¹ | DP² | Mₙ³ | DP³ | M_{w}/Mₙ³ | Mₙ⁴ | DP⁴ | M_{w/}Mₙ⁴ | Tg (°C)⁵ | Fp (°C)⁵ | Zp (°C)⁶ |
|---|---|---|---|---|---|---|---|---|---|---|---|---|
| 1 a | 25 | 22,7 | 22,5 | 10690 | 32,8 | 1,11 | 6821 | 19,3 | 1,07 | 55,4 | - | 290,9 |
| 1 b | 50 | 51,1 | 47,5 | 19630 | 63,7 | 1,11 | 11017 | 33,8 | 1,05 | 58,45 | 152,2 | 283,9 |
| 1 c | 100 | 105 | 93,4 | 39800 | 133,7 | 1,06 | 19929 | 64,8 | 1,04 | 59,8 | 159,4 | 277,8 |

| | | | | | | | | | | | | |
|---|---|---|---|---|---|---|---|---|---|---|---|---|
| ¹ ermittelt aus ¹H-NMR Spektren, 300 MHz, C₂D₂Cl₄ | | | | | | | | | | | | |
| ² ermittelt aus UV/VIS Spektren, Chloroform, ε=4,96*10⁴ 1/mol*cm | | | | | | | | | | | | |
| ³ ermittelt aus GPC-Elugrammen, Eluent: THF, Eichkurve: Polystyrol, 30°C | | | | | | | | | | | | |
| ⁴ ermittelt aus MALDI-TOF-Massenspektren | | | | | | | | | | | | |
| 5) ermittelt aus DSC-Diagramm, zwei Heiz-, eine Kühlkurve, -160°C - 300°C, Heizrate: 10°C/min | | | | | | | | | | | | |
| 6) ermittelt aus TGA-Kurve, 10°C/min, Mittelpunkt | | | | | | | | | | | | |

### 14.1.2 PLLA 2 a-c

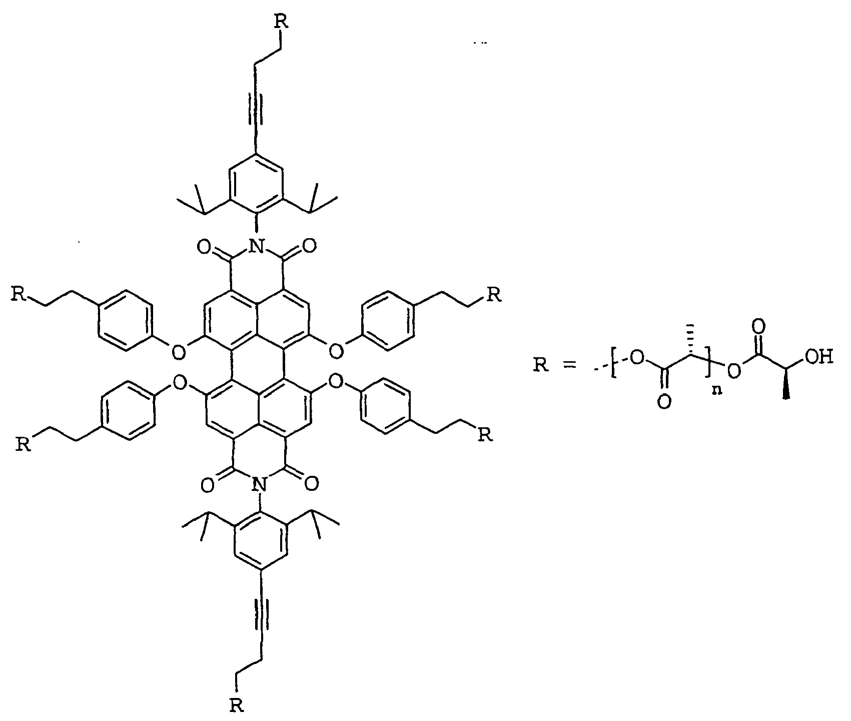

**Tabelle 3**

| Nr. | DP, angestrebt | Initiator | Monomer | Katalysator |
|---|---|---|---|---|
| 2 a | 25 | 200 mg | 1,51 g | 6,7 mg |
| | | 0,14 mmol | 10,5 mol | 0,017 mmol |
| 2 b | 50 | 200 mg | 3,03 g | 6,7 mg |
| | | 0,14 mmol | 21 mol | 0,017 mmol |
| 2 c | 100 | 100 mg | 3,03 g | 3,35 mg |
| | | 0,07 mmol | 21 mol | 0,008 mmol |

Charakterisierung:

**Tabelle 4: Charakterisierung und thermische Eigenschaften der hergestellten PLLA 6Arm Sterne**

| Nr. | DP Ziel | DP¹ | DP² | Mₙ³ | DP³ | M_{w}/Mₙ³ | Mₙ⁴ | DP⁴ | M_{w}/Mₙ⁴ | Tg (°C) ⁵ | Fp (°C) ⁵ | Zp (°C) ⁶ |
|---|---|---|---|---|---|---|---|---|---|---|---|---|
| 2 a | 25 | 24 | 22 | 13560 | 28,1 | 1,12 | 10397 | 20,8 | 1,08 | 49,6 | - | 261,2 |
| 2 b | 50 | 54,7 | 48,5 | 28730 | 63,2 | 1,08 | 20515 | 44,2 | 1,06 | 50,3 | 147,0 | 248,3 |
| 2 c | 100 | 95,4 | 97,2 | 53720 | 121,0 | 1,06 | 38661 | 86,2 | 1,1 | 57,7 | 160,1 | 252,6 |

| | | | | | | | | | | | | |
|---|---|---|---|---|---|---|---|---|---|---|---|---|
| ¹ ermittelt aus ¹H-NMR-Spektren, 300 MHz, C₂D₂Cl₄ | | | | | | | | | | | | |
| ² ermittelt aus UV/VIS-Spektren, Chloroform, ε=4,96*10⁴ 1/mol*cm | | | | | | | | | | | | |
| ³ ermittelt aus GPC-Elugrammen, Eluent: THF, Eichkurve: Polystyrol, 30°C | | | | | | | | | | | | |
| ⁴ ermittelt aus MALDI-TOF-Massenspektren | | | | | | | | | | | | |
| ⁵ ermittelt aus DSC-Diagramm, zwei Heiz-, eine Kühlkurve, -160°C - 300°C, Heizrate: 10°C/min | | | | | | | | | | | | |
| ⁶ ermittelt aus TGA-Kurve, 10°C/min, Mittelpunkt | | | | | | | | | | | | |

### 14.2. Allgemeine Arbeitsvorschrift für ringöffnende lebende Polymerisation von Polycaprolactonen

### Beispielvorschrift:

Das Monomer ε-Caprolacton wird 12 h über Calciumhydrid getrocknet, destilliert und unter N₂ aufbewahrt. Toluol wird über Kalium getrocknet und unter trockenem Argon destilliert.

In ein ausgeheiztes Schlenkrohr werden 225 mg (0,20 mmol) des Initiatorfarbstoffmoleküls eingewogen. Das Schlenkrohr wird mit einem Septum verschlossen, unter Argon gesetzt, 2,28 g (0,02 mol) ε-Caprolacton mit einer Spritze über das Septum zugespritzt, und das Gemisch wird im Ölbad auf 90°C erwärmt. Es werden 6,4 mg (0,016 mmol) Sn(Oct)₂ in Form einer 1 %igen Lösung in wasserfreiem Toluol als Katalysator zugespritzt. Die Menge an Katalysator entspricht 1/50 der molaren Menge der OH-Gruppen. Die Temperatur wird auf 110°C erhöht, und die Mischung wird 48 h gerührt. Das dann feste Polymer wird in CH₂Cl₂ gelöst und durch Eintropfen unter Rühren in MeOH ausgefällt, wobei man das Polymer 3 a als rotes Pulver in quantitativer Ausbeute erhält, das anschließend im Feinvakuum getrocknet wird.

3 b-d und 4 a-d werden analog erhalten, wobei jedoch die in der Tabelle angebenen Mengen von Initiator und Monomer eingesetzt werden.

¹H-NMR-Spektrum des 4Arm Sternpolymers 3 a (300 MHz, C₂D₂Cl₄, 25°C) :
δ (ppm) = 8,16 (s, 4H), 7,36 (tr, 2H), 7,20 (d, 4H), 7,08 (d, 8H), 6,90 (d, 8H), 3,99 (tr, 203H), 3,56 (tr, 8H), 2,26 (tr, 210H), 1,58 (m, 427H), 1,13 (m, 218H), 1,06 (s, 24H)

### Beispiel ¹H-NMR-Spektrum für 6Arm Sternpolymere:

¹H-NMR-Spektrum der Verbindung 4 b (250 MHz, C₂D₂Cl₄, 25°C) :
δ (ppm) = 8,11 (s, 4H), 7,20 (s, 4H), 7,03 (d, 8H), 6,83 (d, 8H), 3,94 (tr, 319,5H), 2,21 (tr, 333,8H), 1,53 (m, 761,7H), 1,28 (m, 348,5H), 1,00 (s, 24H)

### 14.2.1 PCL 3 a-d

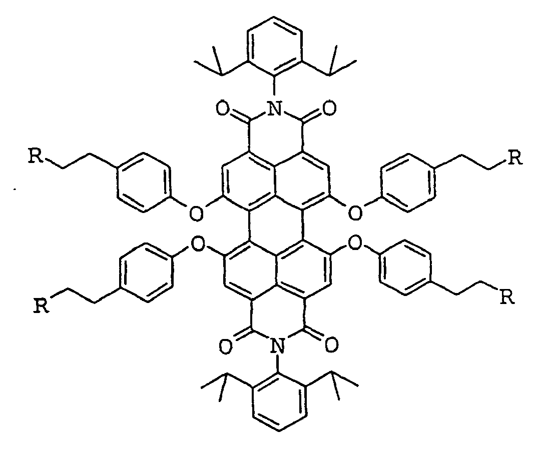

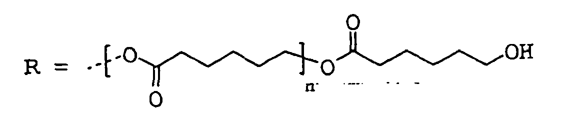

**Tabelle 5**

| Nr. | DP, angestrebt | Initiator | Monomer | Katalysator |
|---|---|---|---|---|
| 3 a | 10 | 11,25 mg | 4,56 g | 0,64 mg |
| | | 0,01 mmol | 0,04 mol | 0,016 mmol |
| 3 b | 25 | 225 mg | 2,28 g | 6,4 mg |
| | | 0,20 mmol | 0,02 mol | 0,016 mmol |
| 3 c | 50 | 225 mg | 4,56 g | 6,4 mg |
| | | 0,20 mmol | 0,04 mol | 0,016 mmol |
| 3 d | 100 | 112,5 mg | 4,56 g | 3,2 mg |
| | | 0,10 mmol | 0,04 mol | 0,008 mmol |

Charakterisierung:

**Tabelle 6: Charakterisierung und thermische Eigenschaften der hergestellten PCL 4Arm Sterne**

| Nr. | DP Ziel | DP¹ | DP² | Mₙ³ | DP³ | M_{w}/Mₙ³ | Mₙ⁴ | DP⁴ | M_{w}/Mₙ⁴ | T_{g} (°C)⁵ | Fp (°C)⁵ | Zp (°C)⁶ |
|---|---|---|---|---|---|---|---|---|---|---|---|---|
| 3 a | 10 | 9,5 | 10,6 | 9739 | 18,6 | 1,13 | 5008 | 8 | 1,08 | -52,6 | 39,36 | 338,1 |
| 3 b | 25 | 25,4 | 25,8 | 19820 | 40,7 | 1,1 | 11728 | 22,9 | 1,09 | - | 52,9 | 338,1 |
| 3 c | 50 | 36,5 | 35,2 | 25660 | 53,4 | 1,08 | 15603 | 31,4 | 1,02 | - | 53,6 | 348,8 |
| 3 d | 100 | 111 | 100,1 | 63000 | 135,2 | 1,07 | 37211 | 78,7 | 1,04 | - | 57,6 | 405,5 |

| | | | | | | | | | | | | |
|---|---|---|---|---|---|---|---|---|---|---|---|---|
| ¹ ermittelt aus ¹H-NMR-Spektren, 300 MHz, C₂D₂Cl₄ | | | | | | | | | | | | |
| ² ermittelt aus UV/VIS-Spektren, Chloroform, ε=4,96*10⁴ 1/mol*cm | | | | | | | | | | | | |
| ³ ermittelt aus GPC-Elugranunen, Eluent: THF, Eichkurve: Polystyrol, 30°C | | | | | | | | | | | | |
| ⁴ ermittelt aus MALDI-TOF-Massenspektren | | | | | | | | | | | | |
| ⁵ ermittelt aus DSC-Diagramm, zwei Heiz-, eine Kühlkurve, -160°C - 300°C, Heizrate: 10°C/min | | | | | | | | | | | | |
| ⁶ ermittelt aus TGA-Kurve, 10°C/min, Mittelpunkt | | | | | | | | | | | | |

### 14.2.2 PCL 4 a-d

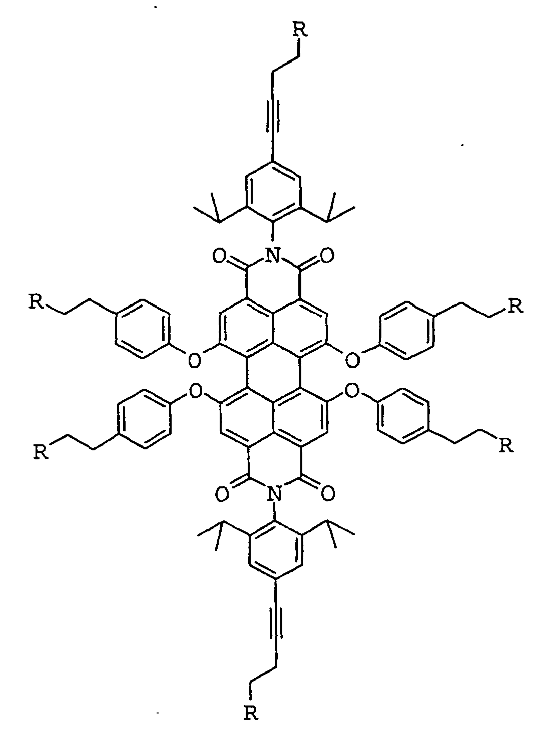

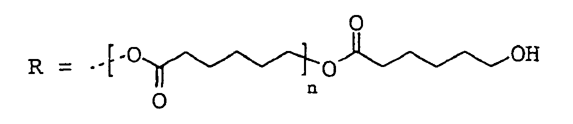

**Tabelle 7**

| Nr. | DP, angestrebt | Initiator | Monomer | Katalysator |
|---|---|---|---|---|
| 4 a | 10 | 10 mg | 0,78 g | 3,36 mg |
| | | 0,007 mmol | 4,2 mmol | 0,008 mmol |
| 4 b | 25 | 200 mg | 2,40 g | 6,72 mg |
| | | 0,14 mmol | 21 mmol | 0,017 mmol |
| 4 c | 50 | 200 mg | 3,03 g | 6,72 mg |
| | | 0,14 mmol | 42 mmol | 0,017 mmol |
| 4 d | 100 | 100 mg | 7,8 g | 3,36 mg |
| | | 0,07 mmol | 42 mmol | 0,008 mmol |

Charakterisierung:

**Tabelle 8: Charakterisierung und thermische Eigenschaften der hergestellten PCL 4Arm Sterne**

| Nr. | DP Ziel | DP¹ | DP² | Mₙ³ | DP³ | M_{w}/Mₙ³ | Mₙ⁴ | DP⁴ | M_{w}/Mₙ⁴ | Tg (°C)⁵ | Fp (°C)⁵ | Zp (°C)⁶ |
|---|---|---|---|---|---|---|---|---|---|---|---|---|
| 4 a | 10 | 10,7 | 8,7 | 7040 | 8,2 | 1,14 | 6669 | 7,7 | 1,09 | -52,7 | 38,5 | 304,2 |
| 4 b | 25 | 26,6 | 24,2 | 22480 | 30,8 | 1,16 | 15464 | 20,5 | 1,07 | - | 50,1 | 331,1 |
| 4 c | 50 | 60,4 | 49,7 | 36200 | 50,8 | 1,07 | 27292 | 37,8 | 1,05 | - | 53,5 | 325,0 |
| 4 d | 100 | 93,2 | 100,4 | 51880 | 73,7 | 1,11 | 41765 | 58,9 | 1,03 | - | 54,7 | 334,2 |

| | | | | | | | | | | | | |
|---|---|---|---|---|---|---|---|---|---|---|---|---|
| 1 ermittelt aus ¹H-NMR Spektren, 300 MHz, C₂D₂Cl₄ | | | | | | | | | | | | |
| ² ermittelt aus UV/VIS Spektren, Chloroform, ε=4,96*10⁴ l/mol*cm | | | | | | | | | | | | |
| ³ ermittelt aus GPC-Elugrammen, Eluent: THF, Eichkurve: Polystyrol, 30°C | | | | | | | | | | | | |
| ⁴ ermittelt aus MALDI-TOF-Massenspektren | | | | | | | | | | | | |
| ⁵ ermittelt aus DSC-Diagramm, zwei Heiz-, eine Kühlkurve, -160°C - 300°C, Heizrate: 10°C/min | | | | | | | | | | | | |
| ⁶ ermittelt aus TGA-Kurve, 10°C/min, Mittelpunkt | | | | | | | | | | | | |

### 14.2.3. PACL 5 a-d

Allgemeine Arbeitsvorschrift: siehe Caprolacton.

Zur Aufarbeitung wird das Reaktionsgemisch auf Methanol gegossen und ca. 1 h gerührt. Die entstandene Suspension wird zentrifugiert, das Lösungsmittel abdekantiert und das verbleibende Polymer im Feinvakuum getrocknet.

5 a-d werden analog erhalten, wobei jedoch die in der Tabelle angebenen Mengen von Initiator und Monomer eingesetzt werden.

¹H-NMR-Spektrum der Verbindung PACL 5 a: (300 MHz, C₂D₂Cl₄, 25°C):
δ (ppm) = 8,12 (s, 4H), 7,32 (tr, 2H), 7,16 (d, 4H), 7,05 (d, 8H), 6,85 (d, 8H), 3,93 (m, 74H), 3,52 (tr, 8H), 2,82 (s, 8H), 2,59 (tr, 4H), 2,24 (m, 74H), 1,74 (tr, 74H), 1,23 (m, 148H), 0,98 (m, 60H), 0,73 (m, 332H)

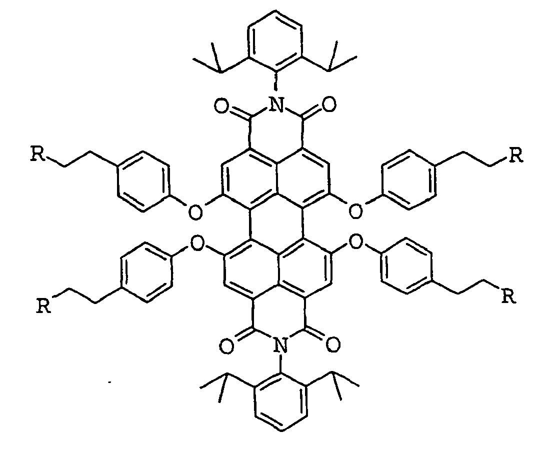

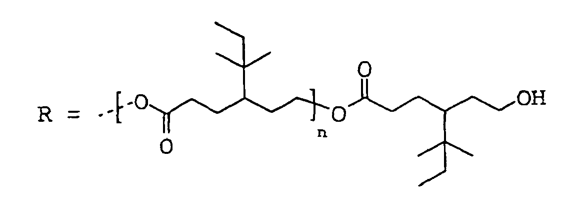

**Tabelle 9**

| Nr. | DP, angestrebt | Initiator | Monomer | Katalysator |
|---|---|---|---|---|
| 5 a | 10 | 200 mg | 1,03 g | 4,48 mg |
| | | 0,14 mmol | 5,6 mmol | 0,012 mmol |
| 5 b | 25 | 200 mg | 2,58 g | 4,48 mg |
| | | 0,14 mmol | 0,014 mol | 0,012 mmol |
| 5 c | 50 | 100 mg | 2,58 g | 2,24 mg |
| | | 0,07 mmol | 0,014 mol | 0,006 mmol |
| 5 d | 100 | 100 mg | 5,16 g | 2,24 mg |
| | | 0,07 mmol | 0,028 mol | 0,006 mmol |

Charakterisierung:

**Tabelle 10: Charakterisierung und thermische Eigenschaften der hergestellten PCL 4Arm Sterne**

| Nr. | DP Ziel | DP¹ | DP² | Mₙ³ | DP³ | M_{w}/Mₙ³ | Mₙ⁴ | DP⁴ | M_{w}/Mₙ⁴ | T_{g} (°C)⁵ | Fp (°C)⁵ | Zp (°C)⁶ |
|---|---|---|---|---|---|---|---|---|---|---|---|---|
| 5-a | 10 | 9,2 | 8,1 | 8224 | 9,5 | 1,14 | 7220 | 7,9 | 1,04 | -13,3 | - | 293,6 |
| 5-b | 25 | 23,7 | 20 | 17670 | 22,3 | 1,16 | 15452 | 19,1 | 1,08 | -21,1 | - | 290,5 |
| 5 c | 50 | 44 | 40,5 | 25280 | 32,6 | 1,07 | 21707 | 27,6 | 1,06 | -24,6 | - | 288,0 |
| 5-d | 100 | 92,1 | 83,2 | 42860 | 55,1 | 1,11 | 33760 | 43,9 | 1,05 | -24,4 | - | 298,7 |

| | | | | | | | | | | | | |
|---|---|---|---|---|---|---|---|---|---|---|---|---|
| ¹ ermittelt aus ¹H-NMR-Spektren, 300 MHz, C₂D₂Cl₄ | | | | | | | | | | | | |
| ² ermittelt aus UV/VIS-Spektren, Chloroform, ε=4,96*10⁴ l/mol*cm | | | | | | | | | | | | |
| ³ ermittelt aus GPC-Elugrammen, Eluent: THF, Eichkurve: Polystyrol, 30°C | | | | | | | | | | | | |
| ⁴ ermittelt aus MALDI-TOF-Massenspektren | | | | | | | | | | | | |
| ⁵ ermittelt aus DSC-Diagramm, zwei Heiz-, eine Kühlkurve, -160°C - 300°C, Heizrate: 10°C/min | | | | | | | | | | | | |
| ⁶ ermittelt aus TGA-Kurve, 10°C/min, Mittelpunkt | | | | | | | | | | | | |

### 14.3. Allgemeine Arbeitsvorschrift für Atom-Transfer-Radikal Polymerisation

In ein ausgeheiztes trockenes Schlenkrohr werden Kupfer(I)bromid (CuBr), 2,2'-Bipyridin (Bpy), Monomer und Initiator N,N'-Bis [4- (butin-1-ol)-2,6-diisopropylphenyl]-1,6,7,12-tetra-[4-(ethyl-2-bromisobuttersäureamid)phenoxy]perylen-3,4,9,10-tetracarbonsäurediimid eingewogen und mit einem Septum verschlossen. Das Verhältnis von [Initiator]:[CuBr]:[bpy] beträgt 1:1:2. Es kann trockenes Toluol als Lösungsmittel hinzugegeben werden.

Das Reaktionsgemisch wird mittels "freeze, pump and thaw" entgast, das heißt, der Inhalt des Schlenkrohres wird zuerst in flüssigem Stickstoff eingefroren, dann wird das Gefäß bis zu einem Druck < 1·10⁻³ mbar evakuiert, und anschließend läßt man bei Raumtemperatur wieder auftauen. Dieser Zyklus wird noch zweimal wiederholt. Das evakuierte Schlenkrohr wird schließlich in einem Ölbad auf 110°C erhitzt und das Reaktionsgemisch wird 48 h bei dieser Temperatur gerührt. Anschließend wird das entstandene Polymer 6 a in CH₂Cl₂ gelöst und durch Eintropfen dieser Lösung unter Rühren in Methanol ausgefällt. Das feine Pulver wird über eine D4-Glasfritte abfiltriert und im Feinvakuum getrocknet.

6 b-c und 7 a-b werden analog erhalten, wobei jedoch die in der Tabelle angebenen Mengen von Initiator und Monomer eingesetzt werden.

¹H-NMR-Spektrum der Verbindung PS 6 a (300 MHz, C₂D₂Cl₄, 25°C):
δ (ppm) = 8,13 (s, 4H), 7,36 (d, 2H), 7,27 (tr, 4H), 7,04 (m, 2701H), 6,56 (m, 1710H), 1,84 (m, 951H), 1,42 (s, 1998H), 1,07 (s, 18H), 0,80 (m, 41H)

### 14.3.1 PS 6 a-c

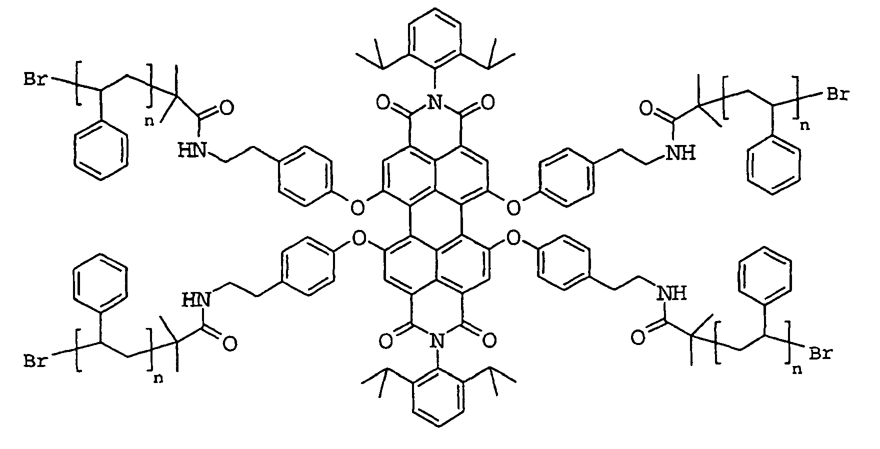

**Tabelle 11**

| Nr. | Initiator | CuBr | Bpy | Monomer | LM | Umsatz |
|---|---|---|---|---|---|---|
| 6 a | 300 mg | 93,0 mg | 0,202 g | 6,76 g | 3 ml | 24,6 % |
| | 0,162 mmol | 0,649 mmol | 1,3 mmol | 64,9 mmol | Toluol | |
| 6 b | 300 mg | 93,0 mg | 0,202 g | 13,52 g | - | 80,1 % |
| | 0,162 mmol | 0,648 mmol | 1,3 mmol | 0,130 mol | | |
| 6 c | 300mg | 93,0 mg | 0,202 g | 6,76 g | - | 70,4 % |
| | 0,162 mmol | 0,649 mmol | 1,3 mmol | 64,9 mmol | | |

Charakterisierung:

**Tabelle 12: Charakterisierung und thermische Eigenschaften der hergestellten PCL 4Arm Sterne**

| Nr. | DP Ziel | DP¹ | Mₙ² | DP² | M_{w}/Mₙ² | Mₙ³ | DP³ | M_{w}/Mₙ³ | Tg (°C)⁴ | Fp (°C)⁴ | Zp (°C)⁵ |
|---|---|---|---|---|---|---|---|---|---|---|---|
| 6 a | 100 | 24,6 | 9594 | 18,6 | 1,24 | 9386 | 18,1 | 1,2 | 102,2 | - | 421,7 |
| 6 b | 200 | 160,2 | 51800 | 120,0 | 1,19 | 67686 | 158 | 1,12 | 107,2 | - | 427,3 |
| 6 c | 100 | 70,4 | 24500 | 54,4 | 1,29 | 29759 | 67 | 1,28 | 14,8 | - | 420,2 |

| | | | | | | | | | | | |
|---|---|---|---|---|---|---|---|---|---|---|---|
| ¹ ermittelt aus UV/VIS-Spektren, Chloroform, ε=4,96*10⁴ 1/mol*cm | | | | | | | | | | | |
| ² ermittelt aus GPC-Elugrammen, Eluent: THF, Eichkurve: Polystyrol, 30°C | | | | | | | | | | | |
| ³ ermitteßt aus MALDI-TOF-Massenspektren | | | | | | | | | | | |
| ⁴ ermittelt aus DSC-Diagramm, zwei Heiz-, eine Kühlkurve, -160°C - 300°C, Heizrate: 10°C/min | | | | | | | | | | | |
| ⁵ ermittelt aus TGA-Kurve, 10°C/min, Mittelpunkt | | | | | | | | | | | |

### 14.3.2 PS 7 a-b

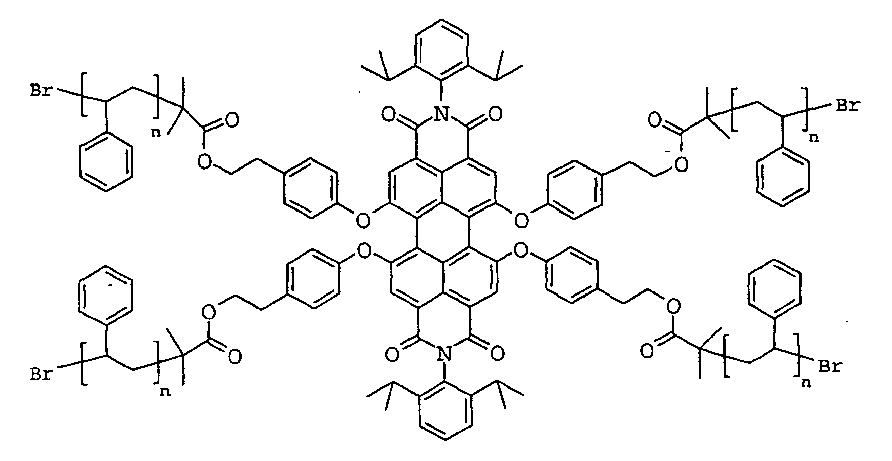

**Tabelle 13**

| Nr. | Initiator | CuBr | Bpy | Monomer | LM | Umsatz |
|---|---|---|---|---|---|---|
| 7 a | 300 mg | 93,0 mg | 0,202 g | 6,76 g | - | 67 % |
| | 0,162 mmol | 0,649 mmol | 1,3 mmol | 64,9 mmol | | |
| 7 b | 300 mg | 93,0 mg | 0,202 g | 3,38 g | - | 60 % |
| | 0,162 mmol | 0,648 mmol | 1,3 mmol | 32,45 mmol | | |

Charakterisierung:

**Tabelle 14: Charakterisierung und thermische Eigenschaften der hergestellten PCL 4Arm Sterne**

| Nr. | DP Ziel | DP¹ | Mₙ² | DP² | M_{w}/Mₙ² | Mₙ³ | DP³ | M_{w}/Mₙ³ | Tg (°C)⁴ | Fp (°C)⁴ | Zp (°C)⁵ |
|---|---|---|---|---|---|---|---|---|---|---|---|
| 7 a | 100 | 67,1 | 23670 | 52,4 | 1,24 | 18095 | 39 | 1,2 | 103,3 | - | 410,5 |
| 7 b | 50 | 29,9 | 11390 | 22,9 | 1,27 | 8513 | 16 | 1,2 | 104,2 | - | 411,6 |

| | | | | | | | | | | | |
|---|---|---|---|---|---|---|---|---|---|---|---|
| ¹ ermittelt aus UV-VIS-Spektren, Chloroform, ε=4,96*10⁴ l/mol*cm | | | | | | | | | | | |
| ² ermittelt aus GPC-Elugrammen, Eluent: THF, Eichkurve: Polystyrol, 30°C | | | | | | | | | | | |
| ³ ermittelt aus MALDI-TOF-Massenspektren | | | | | | | | | | | |
| ⁴ ermittelt aus DSC-Diagramm, zwei Heiz-, eine Kühlkurve, -160°C - 300°C, Heizrate: 10°C/min | | | | | | | | | | | |
| ⁵ ermittelt aus TGA-Kurve, 10°C/min, Mittelpunkt | | | | | | | | | | | |

### Beispiel 2

### Herstellung von sternförmigen fluoreszierenden Polypeptiden

### 1. Polymerisationsexperimente

In mit einem Trocknungsrohr versehenen Schlenkkolben wurde eine Lösung des entsprechenden α-Aminosäure-N-carboxyanhydrids in trockenem DMF (etwa 0,2 g/ml) gegeben. Die verwendeten α-Aminosäure-N-carboxyanhydride γ-Benzyl-L-glutamat-N-carboxyanhydrid (Bn-Glu NCA) und ε-Benzyloxycarbonyl-L-lysin-N-carboxyanhydrid (Z-LysNCA) wurden gemäß der von Poche et al., Syn. Commun. 29 (1999), 843, beschriebenen Vorgehensweise synthetisiert. Dann wurde dem Schlenkkolben eine Lösung der oben erhaltenen Verbindung N,N'-Bis-(2,6-diisopropylphenyl)-1,6,7,12-tetra(4-(2-aminoethyl)phenoxy)perylen-3,4,9,10-tetracarbonsäurediimid in trockenem DMF zugegeben, und das Reaktionsgemisch wurde bei Raumtemperatur gerührt. Die Menge an Initiatorlösung hing von der gewünschten Armlänge des Sternpolymers ab, welche über das molare Verhältnis von α-Aminosäure-N-carboxyanhydrid und Initiator N,N'-Bis-(2,6-diisopropylphenyl)-1,6,7,12-tetra[4-(2-aminoethyl)phenoxy]perylen-3,4,9,10-tetracarbonsäurediimid kontrolliert wurde. Nach 5 Tagen wurde die Lösung langsam einem 20-fachen (vol/vol) Überschuss an Diethylether zugegeben. Das präzipitierte Polymer wurde abfiltriert und vakuumgetrocknet.

Poly(γ-benzyl-L-glutamat)-Sternpolymer (I):
¹H-NMR (500 MHz, C₂D₂Cl₄):
δ = 8,23 (s, 4H; Perylen), 7,25 (b, 6H + 5H x 4n; Ph-H + Bn-H), 7,02 (b, 8H; Ph-H), 6,89 (b, 8H; Ph-H), 5,0 (s, 2H x 4n; Bn-CH₂-), 4,0 (b, 1H x 4n; C_{α}-H), 2,30 (b, 4H x 4n; -CH₂CH₂-), 1,05 (b, 24H; -CH_{3 isopropyl})

Poly(ε-benzyloxycarbonyl-L-lysin)-Sternpolymer (II):
¹H-NMR (500 MHz, C₂D₂Cl₄):
δ = 7,25 (b, 5H x 4n; Bn-H), 5,0 (b, 2H x 4n; Bn-CH₂-), 3,90 (b, 1H x 4n; C_{α}-H), 3,10 (b, 2H x 4n; -CH₂NH-), 2,0 (b, 2H x 4n; -CH₂-), 1,50 (b, 4H x 4n; -CH₂CH₂-), 1,05 (b, 24H, CH_{3 isopropyl})

n gibt die mittlere Anzahl der Monomereinheiten pro Arm an.

### 2. Entfernung der Schutzgruppen vom Poly(γ-benzyl-L-glutamat)-Sternpolymer (I) und vom Poly(ε-benzyloxycarbonyl-L-lysin)-Sternpolymer (II)

Ein vierfacher molarer Überschuss von HBr (33 Gew.-% in AcOH) wurde einer Lösung der Verbindung I oder der Verbindung II in Trifluoressigsäure zugegeben. Das Reaktionsgemisch wurde für 1 h bei Raumtemperatur gerührt. Dann wurde Diethylether zugegeben, und das präzipitierte Polymer wurde ausgiebig mit Diethylether gewaschen. Nach Trocknen unter Vakuum wurden das Poly(L-glutaminsäure)-Sternpolymer (III) und das Poly(L-lysin)-Sternpolymer (IV) in quantitativer Ausbeute erhalten.

Poly(L-glutaminsäure)-Sternpolymer (III):
¹H-NMR (300 MHz, D₂O):
δ = 8,30 (b, 4H; Perylen), 7,30 (b, 6H; Ph-H), 7,13 (b, 8H; Ph-H), 6,94 (b, 8H; Ph-H), 4,00 (b, 1H x 4n; C_{α}-H), 2,20 (b, 4H x 4n;
CH₂CH₂-), 1,03 (b, 24H; -CH_{3 isopropyl})

Poly(L-lysin)-Sternpolymer (IV):
¹H-NMR (300 MHz, D₂O) :
δ = 8,23 (b, 4H; Perylen), 7,45 (b, 6H; Ph-H), 7,15 (b, 8H; Ph-H), 7,04 (b, 8H; Ph-H), 4,30 (t, C_{α}-H; 1H x 4n), 3,00 (t, -CH₂-NH-R; 2H x 4n), 1,70 (b, 4H x 4n; -CH₂CH₂-), 1,40 (b, 2H x 4n, -CHCH₂-), 1,10 (b, 24H; -CH_{3 isopropyl})

## Patentansprüche

1. Verwendung von Perylen-3,4,9,10-tetracarbonsäurediimidverbindungen der Formel (I) 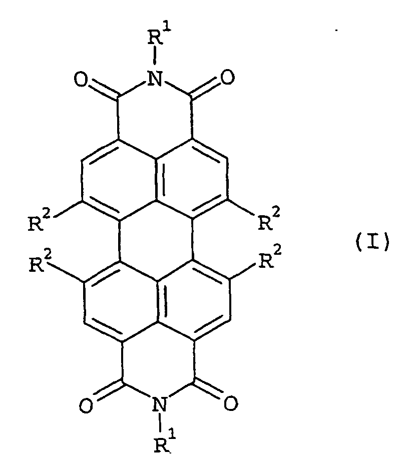
worin R¹ jeweils unabhängig eine Alkyl- oder Arylgruppe ist, die unverzweigt oder verzweigt, unsubstituiert oder substituiert sein kann, und
R² jeweils unabhängig H, Halogen, C₁-C₄-Alkyl, C₁-C₄-Alkoxy, C₅-C₁₅-Aryl, C₅-C₁₅-Aryloxy oder einen Rest darstellt, der mindestens eine funktionelle Gruppe Y enthält, ausgewählt aus Hydroxy, Ether, Ester, Halogen, Amin, Amid, Thiol, ethylenisch ungesättigter Doppelbindung, acetylenisch ungesättigter Dreifachbindung oder/und Carbonsäure, die ggf. mit einer Schutzgruppe oder einer Aktivierungsgruppe versehen ist,
mit der Maßgabe, dass mindestens ein R² eine funktionelle Gruppe Y enthält,
als Initiator oder/und als Reaktionspartner für eine Polymerisationsreaktion.

2. Verwendung nach Anspruch 1,**dadurch gekennzeichnet, dass** die Perylen-3,4,9,10-tetracarbonsäurediimidverbindung der Formel (I) eine Fluoreszenz bei einer Emissionswellenlänge von > 500 nm aufweist.

3. Verwendung nach Anspruch 1 oder 2, **dadurch gekennzeichnet, dass** die Perylen-3,4,9,10-tetracarbonsäurediimidverbindung mindestens eine weitere funktionelle Gruppe Y in einem Rest R¹ enthält.

4. Verwendung nach den Ansprüchen 1 bis 3, **dadurch gekennzeichnet, dass** Y eine gegebenenfalls mit einer Schutzgruppe versehene primäre Amingruppe darstellt.

5. Verwendung nach den Ansprüchen 1 bis 4, **dadurch gekennzeichnet, dass** R² einen Rest 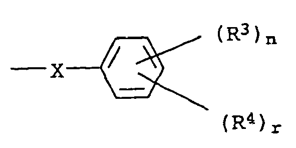 darstellt, worin
X O oder S bedeutet und
R³ einen Rest darstellt, der mindestens eine funktionelle Gruppe Y mit der in Anspruch 1 angegebenen Bedeutung enthält, R⁴ C₁-C₄-Alkyl bedeutet, n eine ganze Zahl von 1 bis 5 ist und r eine ganze Zahl von 0 bis 5 ist, mit der Maßgabe, dass n + r ≤ 5.

6. Verwendung nach Anspruch 5, **dadurch gekennzeichnet, dass** R² einen Rest 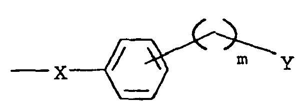 oder 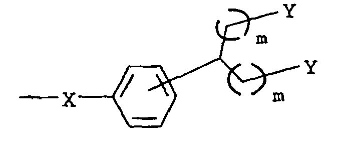 darstellt, worin m für eine ganze Zahl von 1 bis 30 steht und X und Y die in Anspruch 5 angegebene Bedeutung haben.

7. Verwendung nach den Ansprüchen 1 bis 6, **dadurch gekennzeichnet, dass** mindestens zwei funktionelle Gruppen Y in den Resten R² enthalten sind.

8. Verwendung nach den Ansprüchen 1 bis 6, **dadurch gekennzeichnet, dass** mindestens vier funktionelle Gruppen Y in den Resten R² enthalten sind.

9. Verwendung nach den Ansprüchen 1 bis 8, **dadurch gekennzeichnet, dass** mindestens einer der Reste R¹ eine aromatische Gruppe enthält.

10. Verwendung nach Anspruch 9, **dadurch gekennzeichnet, dass** R¹ einen Rest 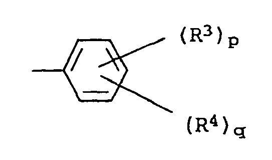 darstellt, worin R³ die in Anspruch 5 angegebene Bedeutung hat R₄ C₁-C₄-Alkyl bedeutet und p und q jeweils eine ganze Zahl von 0 bis 5 darstellen, mit der Maßgabe, dass p + q ≤ 5.

11. Verwendung nach Anspruch 10, **dadurch gekennzeichnet, dass** R¹ einen Rest 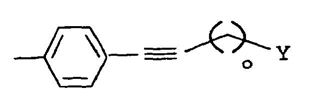 darstellt, worin R³ und Y wie in Anspruch 5 definiert sind und o eine ganze Zahl von 1 bis 30 bedeutet.

12. Farbiges oder/und fluoreszierendes Polymer, **dadurch gekennzeichnet, dass** es durch Umsetzen von mindestens einem Monomeren mit einer Perylen-3,4,9,10-tetracarbonsäurediimidverbindung der Formel (I) 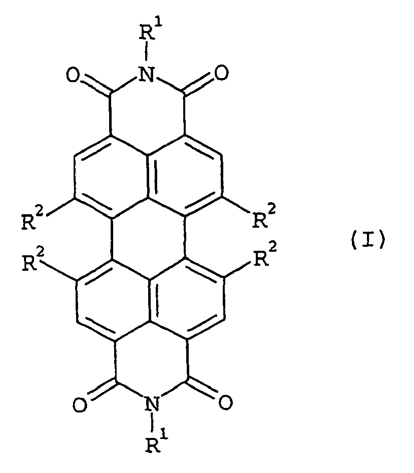
worin R¹ jeweils unabhängig eine Alkyl- oder Arylgruppe ist, die unverzweigt oder verzweigt, unsubstitutiert oder substituiert sein kann, und
R² jeweils unabhängig H, Halogen, C₁-C₄-Alkyl, C₁-C₄-Alkoxy, C₅-C₁₅-Aryl, C₅-C₁₅-Aryloxy oder einen Rest darstellt, der mindestens eine funktionelle Gruppe Y enthält, ausgewählt aus Hydroxy, Ether, Ester, Halogen, Amin, Amid, Thiol, ethylenisch ungesättigter Doppelbindung, acetylenisch ungesättigter Dreifachbindung oder/und Carbonsäure, die gegebenenfalls mit einer Schutzgruppe oder einer Aktivierungsgruppe versehen ist, mit der Maßgabe, dass mindestens ein R² eine funktionelle Gruppe Y enthält,
gebildet ist, wobei die Perylen-3,4,9,10-tetracarbonsäurediimidverbindung kovalent in das Polymer eingebaut ist.

13. Polymer nach Anspruch 12, **dadurch gekennzeichnet, dass** es sich um einen technischen Kunststoff handelt.

14. Polymer nach Anspruch 12 oder 13, **dadurch gekennzeichnet, dass** es sich um ein Polystyrol, Polymethacrylat, Polyacrylat, Silicon oder eine Kombination von diesen handelt.

15. Polymer nach Anspruch 12, **dadurch gekennzeichnet, dass** es sich um einen aliphatischen Polyester, insbesondere ein Polylactid oder Polycaprolacton, ein Polypeptid oder eine Kombination von diesen handelt.

16. Polymer nach Anspruch 12, **dadurch gekennzeichnet, dass** es wasserlösliche Polymerketten umfasst.

17. Polymer nach Anspruch 16, **dadurch gekennzeichnet, dass** die wasserlöslichen Polymerketten ausgewählt werden aus Polypeptiden oder Polyalkylenoxiden.

18. Verwendung eines farbigen oder/und fluoreszierenden Polymers gemäß den Ansprüchen 12 bis 17 in Fluoreszenzsolarkollektoren, als Laserfarbstoff, in Gewächshausfolien, für optische oder/und optoelektronische Anwendungen, als Fluoreszenzlabel oder Fluoreszenzsonde.

19. Sternförmiges Polypeptid, **dadurch gekennzeichnet, dass** es durch Umsetzung von Aminosäuren mit einer Perylen-3,4,9,10-tetracarbonsäurediimidverbindung der Formel (I) 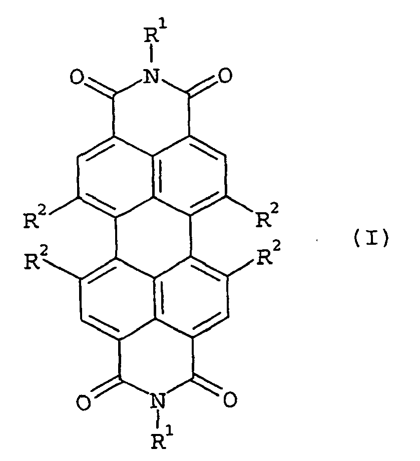
worin R¹ jeweils unabhängig eine Alkyl- oder Arylgruppe ist, die unverzweigt oder verzweigt, unsubstituiert oder substituiert sein kann, und
R₂ jeweils unabhängig H, Halogen, C₁-C₄-Alkyl, C₁-C₄-Alkoxy, C₅-C₁₅-Aryl, C₅-C₁₅-Aryloxy oder einen Rest darstellt, der mindestens eine funktionelle Gruppe Y enthält, ausgewählt aus Hydroxy, Ether, Ester, Halogen, Amin, Amid, Thiol, ethylenisch ungesättigter Doppelbindung, acetylenisch ungesättigter Dreifachbindung oder/und Carbonsäure, die ggf. mit einer Schutzgruppe oder einer Aktivierungsgruppe versehen ist, mit der Maßgabe, dass mindestens ein R² eine funktionelle Gruppe Y enthält,
gebildet ist und in einer wässrigen Lösung fluoreszierend ist.

20. Perylen-3,4,9,10-tetracarbonsäurediimidverbindung der Formel (I) 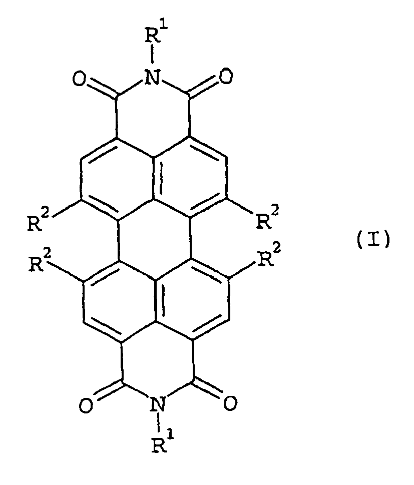
worin R¹ einen Rest darstellt, der mindestens einen aromatischen Ring enthält, und
R² jeweils unabhängig H, Halogen, C₁-C₄-Alkyl, C₁-C₄-Alkoxy, C₅-C₁₅-Aryl, C₅-C₁₅-Aryloxy oder einen Rest darstellt, der mindestens eine funktionelle Gruppe Y enthält, ausgewählt aus Hydroxy, Ether, Ester, Amin, Amid, Thiol, ethylenisch ungesättigter Doppelbindung, acetylenisch ungesättigter Dreifachbindung oder/und Carbonsäure, die gegebenenfalls mit einer Schutzgruppe oder einer Aktivierungsgruppe versehen ist, mit der Maßgabe, dass mindestens drei funktionelle Gruppen Y in der Verbindung vorliegen.

21. Perylen-3,4,9,10-tetracarbonsäurediimidverbindung, welche insbesondere als Zwischenprodukt zur Herstellung einer Verbindung nach Anspruch 20 oder zur Herstellung einer in Anspruch 1 verwendeten Verbindung eingesetzt werden kann, mit der Formel (I) 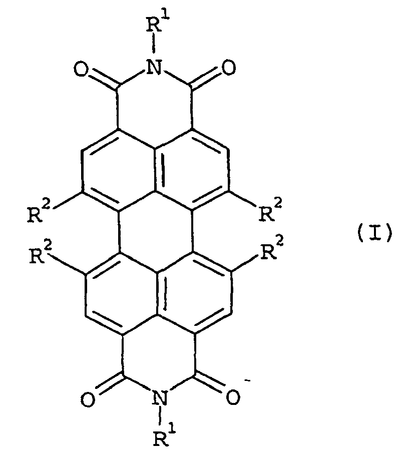
worin R¹ jeweils unabhängig eine Alkyl- oder Arylgruppe ist, die unverzweigt oder verzweigt, unsubstituiert oder substituiert sein kann, mit der Maßgabe, dass mindestens ein R¹ ein Halogen, insbesondere Br, enthält, und
R₂ jeweils unabhängig H, Halogen, C₁-C₄-Alkyl, C₁-C₄-Alkoxy, C₅-C₁₅-Aryl, C₅-C₁₅-Aryloxy oder einen Rest darstellt, der mindestens eine funktionelle Gruppe Y enthält, ausgewählt aus Hydroxy, Ether, Ester, Halogen, Amin, Amid, Thiol, ethylenisch ungesättigter Doppelbindung, acetylenisch ungesättigter Dreifachbindung oder/und Carbonsäure, die gegebenenfalls mit einer Schutzgruppe oder einer Aktivierungsgruppe versehen ist, mit der Maßgabe, dass mindestens ein R² eine funktionelle Gruppe Y enthält.

## Claims

1. The use of perylene-3,4,9,10-tetracarboxylic acid diimide compounds of the formula (I) 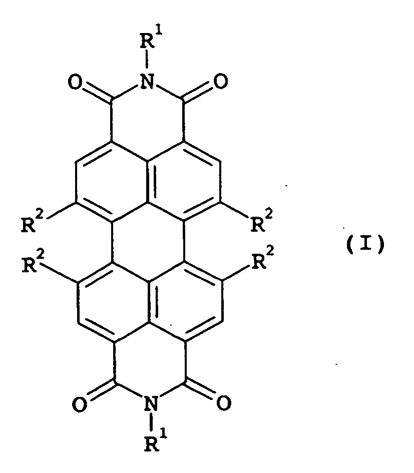
in which each R¹ is, independently of the others, an alkyl or aryl group, which may be unbranched or branched, unsubstituted or substituted, and
each R² is, independently of the others, H, halogen, C₁-C₄-alkyl, C₁-C₄-alkoxy, C₅-C₁₅-aryl, C₅-C₁₅-aryloxy or a radical which comprises at least one functional group Y selected from hydroxyl, ether, ester, halogen, amine, amide, thiol, an ethylenically unsaturated double bond, an acetylenically unsaturated triple bond and/or carboxyl, which may, if appropriate, be provided with a protecting group or an activating group,
with the proviso that at least one R² comprises a functional group Y,
as initiator and/or co-reactant for a polymerization reaction.

2. The use according to claim 1, wherein the perylene-3,4,9,10-tetracarboxylic acid diimide compound of the formula (I) exhibits fluorescence at an emission wavelength of > 500 nm.

3. The use according to claim 1 or 2, wherein the perylene-3,4,9,10-tetracarboxylic acid diimide compound comprises at least one further functional group Y in the radical R¹.

4. The use according to any one of claims 1 to 3, wherein Y is a primary amino group, which is optionally provided with a protecting group.

5. The use according to any one of claims 1 to 4, wherein R² is a radical 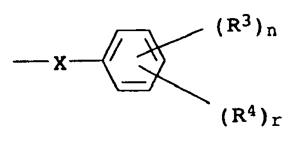
where
X is O or S, and
R³ is a radical which comprises at least one functional group Y as defined in claim 1,
R⁴ is C₁-C₄-alkyl, n is an integer from 1 to 5, and r is an integer from 0 to 5, with the proviso that n + r ≤ 5.

6. The use according to claim 5, wherein R² is a radical 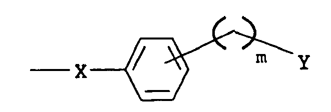 or 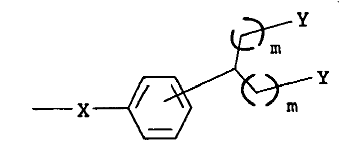
where m is an integer from 1 to 30, and X and Y are as defined in claim 5.

7. The use according to any one of claims 1 to 6, wherein the radicals R² comprise at least two functional groups Y.

8. The use according to any one of claims 1 to 6, wherein the radicals R² comprise at least four functional groups Y.

9. The use according to any one of claims 1 to 8, wherein at least one of the radicals R¹ comprises an aromatic group.

10. The use according to claim 9, wherein R¹ is a radical 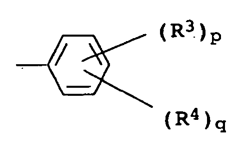
where R³ is as defined in claim 5,
R⁴ is C₁-C₄-alkyl, and p and q are each an integer from 0 to 5, with the proviso that p + q ≤ 5.

11. The use according to claim 10, wherein R¹ is a radical 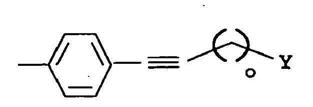
where R³ and Y are as defined in claim 5, and o is an integer from 1 to 30.

12. A colored and/or fluorescent polymer which has been formed by reaction of at least one monomer with a perylene-3,4,9,10-tetracarboxylic acid diimide compound of the formula (I) 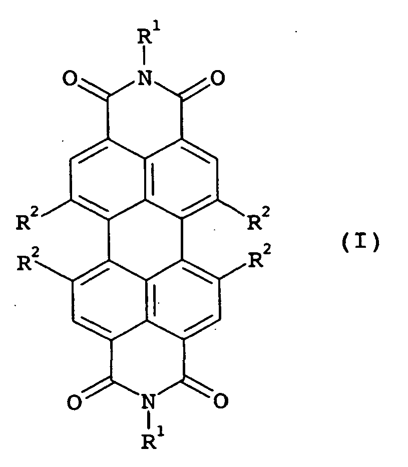
in which each R¹ is, independently of the others, an alkyl or aryl group, which may be unbranched or branched, unsubstituted or substituted, and
each R² is, independently of the others, H, halogen, C₁-C₄-alkyl, C₁-C₄-alkoxy, C₅-C₁₅-aryl, C₅-C₁₅-aryloxy or a radical which comprises at least one functional group Y selected from hydroxyl, ether, ester, halogen, amine, amide, thiol, an ethylenically unsaturated double bond, an acetylenically unsaturated triple bond and/or carboxyl, which may, if appropriate, be provided with a protecting group or an activating group, with the proviso that at least one R² comprises a functional group Y,
where the perylene-3,4,9,10-tetracarboxylic acid diimide compound is covalently bonded into the polymer.

13. A polymer according to claim 12, which is an engineering plastic.

14. A polymer according to claim 12 or 13, which is a polystyrene, polymethacrylate, polyacrylate, silicone or a combination thereof.

15. A polymer according to claim 12, which is an aliphatic polyester, in particular a polylactide or polycaprolactone, a polypeptide or a combination thereof.

16. A polymer according to claim 12, which comprises water-soluble polymer chains.

17. A polymer according to claim 16, wherein the water-soluble polymer chains are selected from polypeptides and polyalkylene oxides.

18. The use of a colored and/or fluorescent polymer according to any one of claims 12 to 17 in fluorescent solar collectors, as laser dye, in greenhouse sheeting, for optical and/or opto-electronic applications, as fluorescent label or fluorescent probe.

19. A star-shaped polypeptide which has been formed by reaction of amino acids with a perylene-3,4,9,10-tetracarboxylic acid diimide compound of the formula (I) 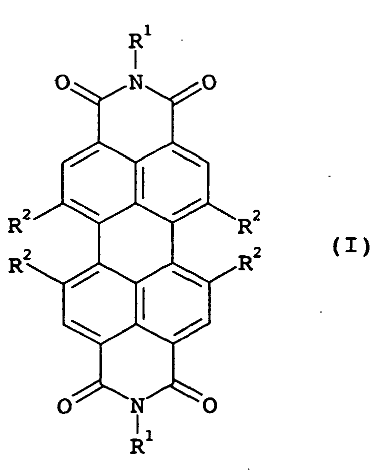
in which each R¹ is, independently of the others, an alkyl or aryl group, which may be unbranched or branched, unsubstituted or substituted, and
each R² is, independently of the others, H, halogen, C₁-C₄-alkyl, C₁-C₄-alkoxy, C₅-C₁₅-aryl, C₅-C₁₅-aryloxy or a radical which comprises at least one functional group Y selected from hydroxyl, ether, ester, halogen, amine, amide, thiol, an ethylenically unsaturated double bond, an acetylenically unsaturated triple bond and/or carboxyl, which may, if appropriate, be provided with a protecting group or an activating group, with the proviso that at least one R² comprises a functional group Y,
and is fluorescent in an aqueous solution.

20. A perylene-3,4,9,10-tetracarboxylic acid diimide compound of the formula (I) 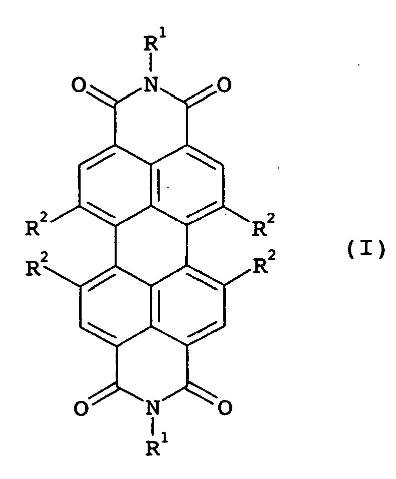
in which R¹ is a radical which comprises at least one aromatic ring, and
each R² is, independently of the others, H, halogen, C₁-C₄-alkyl, C₁-C₄-alkoxy, C₅-C₁₅-aryl, C₅-C₁₅-aryloxy or a radical which comprises at least one functional group Y selected from hydroxyl, ether, ester, amine, amide, thiol, an ethylenically unsaturated double bond, an acetylenically unsaturated triple bond and/or carboxyl, which may, if appropriate, be provided with a protecting group or an activating group, with the proviso that at least three functional groups Y are present in the compound.

21. A perylene-3,4,9,10-tetracarboxylic acid diimide compound which can be employed, in particular, as intermediate in the preparation of a compound according to claim 20 or in the preparation of a compound used in claim 1, having the formula (I) 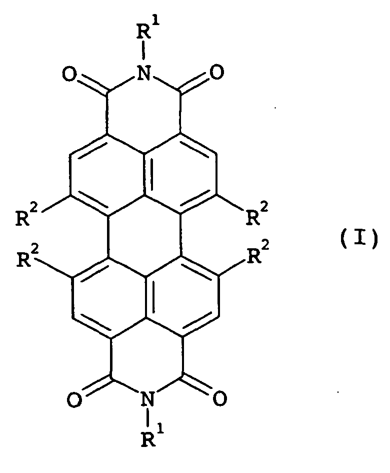
in which each R¹ is, independently of the others, an alkyl or aryl group, which may be unbranched or branched, unsubstituted or substituted, with the proviso that at least one R¹ comprises a halogen, in particular Br, and
each R² is, independently of the others, H, halogen, C₁-C₄-alkyl, C₁-C₄-alkoxy, C₅-C₁₅-aryl, C₅-C₁₅-aryloxy or a radical which comprises at least one functional group Y selected from hydroxyl, ether, ester, halogen, amine, amide, thiol, an ethylenically unsaturated double bond, an acetylenically unsaturated triple bond and/or carboxyl, which may, if appropriate, be provided with a protecting group or an activating group, with the proviso that at least one R² comprises a functional group Y.

## Revendications

1. Utilisation de composés de diimide d'acide pérylène-3,4,9,10-tétracarboxylique de la formule (I) : 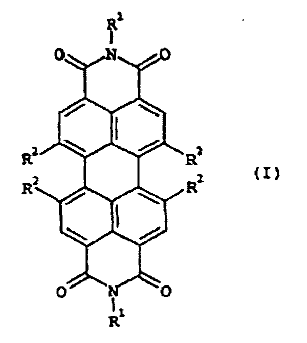
dans laquelle R¹ représente chaque fois indépendamment un groupe alkyle ou aryle qui peut être ramifié ou non ramifié, substitué ou non substitué, et
R² représente chaque fois indépendamment H, de l'halogène, un groupe alkyle en C₁-C₄, alcoxy en C₁-C₄, aryle en C₅-C₁₅, aryloxy en C₅-C₁₅ ou un radical qui contient au moins un groupe fonctionnel Y choisi parmi de l'halogène, un groupe hydroxy, éther, ester, amine, amide, thiol, une double liaison éthyléniquement insaturée, une triple liaison acétyléniquement insaturée et/ou de l'acide carboxylique qui est éventuellement pourvu d'un groupe de protection ou d'un groupe d'activation,
à la condition qu'au moins un R² contienne un groupe fonctionnel Y,
comme amorceur et/ou partenaire réactionnel d'une réaction de polymérisation.

2. Utilisation suivant la revendication 1, **caractérisée en ce que** le composé de diimide d'acide pérylène-3,4,9,10-tétracarboxylique de la formule (I) présente une fluorescence à une longueur d'ondes d'émission de >500 nm.

3. Utilisation suivant la revendication 1 ou 2, **caractérisée en ce que** le composé de diimide d'acide pérylène-3,4,9,10-tétracarboxylique contient au moins un autre groupe fonctionnel Y dans un radical R¹.

4. Utilisation suivant les revendications 1 à 3, **caractérisée en ce que** Y représente un groupe amine primaire éventuellement pourvu d'un groupe de protection.

5. Utilisation suivant les revendications 1 à 4, **caractérisée en ce que** R² représente un radical 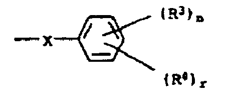
dans lequel
X représente O ou S, et
R³ représente un radical qui contient au moins un groupe fonctionnel Y avec la signification indiquée dans la revendication 1,
R⁴ représente un groupe alkyle en C₁-C₄, n est un nombre entier de 1 à 5, et r est un nombre entier de 0 à 5, à la condition que n + r ≤ 5.

6. Utilisation suivant la revendication 5, **caractérisée en ce que** R² représente un radical 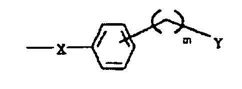
ou 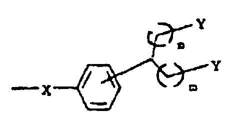
où m représente un nombre entier de 1 à 30 et X et Y ont la signification indiquée dans la revendication 5.

7. Utilisation suivant les revendications 1 à 6, **caractérisée en ce qu'**au moins deux groupes fonctionnels Y sont contenus dans les radicaux R².

8. Utilisation suivant les revendications 1 à 6, **caractérisée en ce qu'**au moins quatre groupes fonctionnels Y sont contenus dans les radicaux R².

9. Utilisation suivant les revendications 1 à 8, **caractérisée en ce qu'**au moins un des radicaux R¹ contient un groupe aromatique.

10. Utilisation suivant la revendication 9, **caractérisée en ce que** R¹ représente un radical 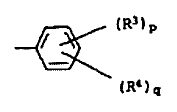
dans lequel R³ a la signification indiquée dans la revendication 5, R⁴ représente un groupe alkyle en C₁-C₄ et p et q représentent chacun un nombre entier de 0 à 5, avec la condition que p + q ≤ 5.

11. Utilisation suivant la revendication 10, **caractérisée en ce que** R¹ représente un radical 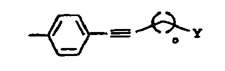
dans lequel R³ et Y sont définis comme dans la revendication 5 et o représente un nombre entier de 1 à 30.

12. Polymère coloré et/ou fluorescent, **caractérisé en ce qu'**il est formé par réaction d'au moins un monomère avec un composé de diimide d'acide pérylène-3,4,9,10-tétracarboxylique de la formule (I) : 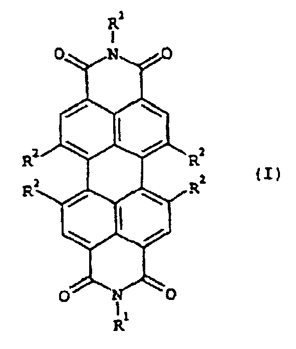
dans laquelle R¹ représente chaque fois indépendamment un groupe alkyle ou aryle qui peut être ramifié ou non ramifié, substitué ou non substitué, et
R² représente chaque fois indépendamment H, de l'halogène, un groupe alkyle en C₁-C₄, alcoxy en C₁-C₄, aryle en C₅-C₁₅, aryloxy en C₅-C₁₅ ou un radical qui contient au moins un groupe fonctionnel Y choisi parmi de l'halogène ou un groupe hydroxy, éther, ester, amine, amide, thiol, une double liaison éthyléniquement insaturée, une triple liaison acétyléniquement insaturée et/ou de l'acide carboxylique qui est éventuellement pourvu d'un groupe de protection ou d'un groupe d'activation,
à la condition qu'au moins un R² contienne un groupe fonctionnel Y,
le composé de diimide d'acide pérylène-3,4,9,10-tétracarboxylique étant incorporé de manière covalente dans le polymère.

13. Polymère suivant la revendication 12, **caractérisé en ce qu'**il s'agit d'une substance synthétique technique.

14. Polymère suivant la revendication 12 ou 13, **caractérisé en ce qu'**il s'agit d'un polystyrène, d'un polyméthacrylate, d'un polyacrylate, d'un silicone ou d'une combinaison de ceux-ci.

15. Polymère suivant la revendication 12, **caractérisé en ce qu'**il s'agit d'un polyester aliphatique, en particulier d'un polylactide ou d'une polycaprolactone, d'un polypeptide ou d'une combinaison de ces derniers.

16. Polymère suivant la revendication 12, **caractérisé en ce qu'**il comporte des chaînes de polymère solubles dans l'eau.

17. Polymère suivant la revendication 16, **caractérisé en ce que** les chaînes de polymère solubles dans l'eau sont choisies parmi des polypeptides ou des oxydes de polyalkylène.

18. Utilisation d'un polymère coloré et/ou fluorescent suivant les revendications 12 à 17 dans des collecteurs solaires fluorescents, comme colorant laser, dans des feuilles pour serres, pour des applications optiques et/ou optoélectroniques, comme étiquette fluorescente ou sonde fluorescente.

19. Polypeptide étoilé, **caractérisé en qu'**il est formé par réaction d'acides aminés avec un composé de diimide d'acide pérylène-3,4,9,10-tétracarboxylique de la formule (I) : 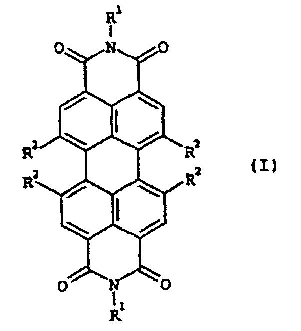
dans laquelle R¹ représente chaque fois indépendamment un groupe alkyle ou aryle qui peut être ramifié ou non ramifié, substitué ou non substitué, et
R² représente chaque fois indépendamment H, de l'halogène, un groupe alkyle en C₁-C₄, alcoxy en C₁-C₄, aryle en C₅-C₁₅, aryloxy en C₅-C₁₅ ou un radical qui contient au moins un groupe fonctionnel Y choisi parmi de l'halogène, un groupe hydroxy, éther, ester, amine, amide, thiol, une double liaison éthyléniquement insaturée, une triple liaison acétyléniquement insaturée et/ou de l'acide carboxylique qui est éventuellement pourvu d'un groupe de protection ou d'un groupe d'activation, à la condition qu'au moins un R² contienne un groupe fonctionnel Y,
et est fluorescent dans une solution aqueuse.

20. Composé de diimide d'acide pérylène-3,4,9,10-tétracarboxylique de la formule (I) : 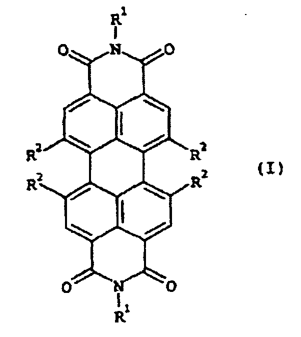
dans laquelle R¹ représente un radical qui contient au moins un noyau aromatique, et
R² représente chaque fois indépendamment H, de l'halogène, un groupe alkyle en C₁-C₄, alcoxy en C₁-C₄, aryle en C₅-C₁₅, aryloxy en C₅-C₁₅ ou un radical qui contient au moins un groupe fonctionnel Y choisi parmi un groupe hydroxy, éther, ester, amine, amide, thiol, une double liaison éthyléniquement insaturée, une triple liaison acétyléniquement insaturée et/ou de l'acide carboxylique qui est éventuellement pourvu d'un groupe de protection ou d'un groupe d'activation, à la condition qu'au moins trois groupes fonctionnels Y soient présents dans le composé.

21. Composé de diimide d'acide pérylène-3,4,9,10-tétracarboxylique, qui peut être mis en oeuvre en particulier comme produit intermédiaire pour la préparation d'un composé suivant la revendication 20 ou pour la préparation d'un composé utilisé dans la revendication 1, et qui répond à la formule (I) : 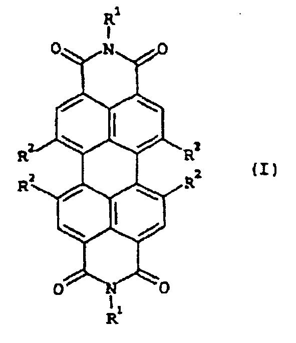
dans laquelle R¹ représente chaque fois indépendamment un groupe alkyle ou aryle, qui peut être ramifié ou non ramifié, substitué ou non substitué, à la condition qu'au moins un R¹ contienne un halogène, en particulier Br, et
R² représente chaque fois indépendamment H, de l'halogène, un groupe alkyle en C₁-C₄, alcoxy en C₁-C₄, aryle en C₅-C₁₅, aryloxy en C₅-C₁₅ ou un radical qui contient au moins un groupe fonctionnel Y choisi parmi de l'halogène, un groupe hydroxy, éther, ester, amine, amide, thiol, une double liaison éthyléniquement insaturée, une triple liaison acétyléniquement insaturée et/ou de l'acide carboxylique qui est éventuellement pourvu d'un groupe de protection ou d'un groupe d'activation, à la condition qu'au moins un R² contienne un groupe fonctionnel Y.
